# EUROPEAN PATENT SPECIFICATION

(11) **EP 2 217 329 B1**
(45) Date of publication and mention of the grant of the patent: **10.01.2018**
(21) Application number: 08847846.6
(22) Date of filing: 07.11.2008
(51) Int. Cl.: A61K 35/51, A61P 43/00, A61K 35/18, A61K 38/18, A61K 35/50

(54) **USE OF UMBILICAL CORD BLOOD IN THE TREATMENT OF PREMATURE BIRTH COMPLICATIONS**
VERWENDUNG VON NABELSCHNURBLUT BEI DER BEHANDLUNG VON FRÜHGEBURTSKOMPLIKATIONEN
UTILISATION DE SANG PROVENANT DU CORDON OMBILICAL POUR LE TRAITEMENT DE COMPLICATIONS LIÉES À LA NAISSANCE PRÉMATURÉE.

(30) Priority: 07.11.2007 US 2375 P
(43) Date of publication of application: 18.08.2010
(60) Divisional application: 17202323.6
(73) Proprietor: Anthrogenesis Corporation, Warren, NJ 07059 (US)
(72) Inventor: HEIDARAN, Mohammad, A., Potomac MD 20854 (US); JOHNSON, Kristine, Erikson, Basking Ridge NJ 07620 (US); HARIRI, Robert, J., Bernardsville NJ 07924 (US)
(74) Representative: Weber, Martin
(86) International application number: PCT/US2008/012540
(87) International publication number: WO 2009/061447

(56) References cited:
- WO-A-02/064755
- WO-A-03/068937
- WO-A-2007/079183
- US-A- 5 356 373
- BALLIN A ET AL: "Autologous umbilical cord blood transfusion." ARCHIVES OF DISEASE IN CHILDHOOD. FETAL AND NEONATAL EDITION NOV 1995, vol. 73, no. 3, November 1995 (1995-11), pages F181-F183, XP002554854 ISSN: 1359-2998
- VAWDA ET AL: "Stem cell therapies for perinatal brain injuries" SEMINARS IN FETAL AND NEONATAL MEDICINE, ELSEVIER, GB, vol. 12, no. 4, 7 June 2007 (2007-06-07), pages 259-272, XP022111605 ISSN: 1744-165X
- HUME ET AL: "Red blood cell transfusions for preterm infants: The role of evidence-based medicine" SEMINARS IN PERINATOLOGY, W.B. SAUNDERS, GB, vol. 21, no. 1, 1 February 1997 (1997-02-01), pages 8-19, XP005437787 ISSN: 0146-0005
- WIDNESS ET AL: "Recombinant erythropoietin in treatment of the premature newborn" SEMINARS IN NEONATOLOGY, SAUNDERS, PHILADELPHIA, US, vol. 3, no. 2, 1 May 1998 (1998-05-01), pages 163-171, XP005831969 ISSN: 1084-2756
- RAO ET AL: "Iron in fetal and neonatal nutrition" SEMINARS IN FETAL AND NEONATAL MEDICINE, ELSEVIER, GB, vol. 12, no. 1, 18 January 2007 (2007-01-18), pages 54-63, XP005832390 ISSN: 1744-165X
- RAMASETHU J ET AL: "Red blood cell transfusions in the newborn" SEMINARS IN NEONATOLOGY, SAUNDERS, PHILADELPHIA, US, vol. 4, no. 1, 1 February 1999 (1999-02-01), pages 5-16, XP004979977 ISSN: 1084-2756

## Description

This application claims the benefit of U.S. Provisional Patent Application Serial No. 61/002,375, filed November 7, 2007.

### 1. FIELD OF THE INVENTION

The present invention relates to compositions and methods for treating one or more disorders or conditions in infants, including premature infants, by administering to such infants umbilical cord blood and, optionally, placental stem cells and/or blood additives.

### 2. BACKGROUND OF THE INVENTION

A full term infant spends 37 to 42 weeks in the uterus. An infant born earlier than 37 weeks is considered premature or preterm. Premature birth is the leading cause of death in the first month of life and is a major public concern. According to the March of Dimes Birth Defects Foundation, in 2002 there were 480,812 premature births (representing 12.1 % of all live births) in the United States, and the cost for medical care of premature infants was 15.5 billion dollars.

Risk factors for preterm labor and delivery include age of the mother (less than 18 years of age or grater than 35 years of age), infection, diabetes mellitus, hypertension, smoking, a pregnancy with multiple fetuses and substance abuse.

Currently, survival rates for infants born from about 23 to about 25 weeks of gestation vary from about 20 percent for infants of 23 gestational weeks, to about 65 percent for infants of 25 gestational weeks. About one third of surviving babies in this age group develop normally; about one third develop mild or moderate disabilities; and about one third develop severe disabilities.

Survival rates for premature infants born from about 26 to about 29 weeks of gestation are about 75 percent for infants born at 26 gestation weeks and about 85 percent for infants born at 29 gestation weeks. About 40 percent of such premature infants who survive develop normally, while about 40 percent develop mild or moderate disabilities and about 20 percent develop one or more severe disabilities.

90 to 95 percent of premature infants born from about 30 to about 33 weeks of gestation survive. About 65% of these premature infants develop normally, while about 20 percent develop mild or moderate disabilities and about 15 percent of these premature infants develop one or more severe disabilities.

Although premature infants born from about 34 to about 37 weeks of gestation are less mature than full-term infants, their survival rate (around 95 percent) is nearly identical to the survival rate for full-term infants, and their long-term prospects are as good as those of any full-term infant.

Physical features of a premature infant include, for example, small size; low birth weight; irregular breathing; and underdeveloped organs or systems such as lung, immune system and brain. Common problems associated with premature infants include but are not limited to anemia, low blood pressures, hyperbilirubinemia, infection, retinopathy, respiratory distress and incomplete development of certain organs, such as lung, eye, immune system, brain, heart, liver and kidney.

Various treatment options, having varying degrees of success, are available to treat disorders and conditions associated with premature infants. For example, premature infants with anemia may be treated with blood transfusions and/or iron supplementation, and surfactant administration is used to treat respiratory distress syndrome associated with preterm birth. No treatment options exist, however, for incomplete organ development. Despite progress, a need continues to exist for development of treatments for disorders and conditions associated with premature infants.

### 3. SUMMARY OF THE INVENTION

The present invention generally relates to umbilical cord blood comprising placental stem cells for use in methods of treating disorders and conditions of a premature infant caused by or associated with premature birth.

Specifically, the present invention provides umbilical cord blood comprising placental stem cells for use in a method of treating a disorder or condition in a premature infant, wherein said disorder or condition is caused by or associated with incomplete development of the brain, wherein said placental stem cells comprise cells that are CD34⁻, CD73⁺, CD105⁺, and CD200⁺.

According to one embodiment, the umbilical cord further comprising a blood additive, wherein said blood additive is erythropoietin, an iron supplement, a vitamin, or red blood cells from a source other than cord blood.

According to another embodiment, said blood additive is erythropoietin.

According to a further embodiment, said erythropoietin is recombinant erythropoietin genetically engineered to increase serum half-life compared to native erythropoietin.

According to another embodiment, said blood additive is a vitamin.

According to a further embodiment, said vitamin is riboflavin (vitamin B2), pyridoxine (vitamin B6), folic acid, vitamin B12, or vitamin E.

According to another embodiment, said blood additive is an iron supplement.

According to a further embodiment, said iron supplement is elemental iron.

According to another embodiment, said blood additive is red blood cells not obtained from cord blood.

According to a further embodiment, said red blood cells have been irradiated with at least 2500 cGy of radiation, or wherein said red blood cells have been leukodepleted.

According to a further embodiment, said premature infant has undergone from 23 to 25 weeks of gestation at birth, from 26 to 29 weeks of gestation at birth, from 30 to 33 weeks of gestation at birth, or from 34 to 37 weeks of gestation at birth.

According to a further embodiment, said premature infant weighs 800 grams or more at birth, 500 grams to 800 grams at birth, or less than 500 grams at birth.

According to a further embodiment, said umbilical cord blood is autologous to the premature infant.

According to a further embodiment, said placental stem cells are autologous to the premature infant.

According to a further embodiment, said umbilical cord blood is obtained from a post-partum mammalian placenta of a full-term birth.

According to a further embodiment, said umbilical cord blood is obtained from a post-partum mammalian placenta of a premature birth.

According to a further embodiment, said placenta is the placenta of said premature infant, the placenta of an infant born at 26 to 29 weeks of gestation, the placenta of an infant born at 30 to 33 weeks of gestation, or the placenta of an infant born at 34 to 37 weeks of gestation.

According to a further embodiment, said umbilical cord blood is obtained from a cord blood bank.

According to a further embodiment, said placental stem cells are stem cells isolated from placental perfusate prior to said use.

According to a further embodiment, said placental stem cells are stem cells contained within placental perfusate.

According to a further embodiment, said placental stem cells are obtained from a post-partum mammalian placenta of a full-term birth, from a post-partum mammalian placenta of a premature birth, or from a placenta of said premature infant.

According to a further embodiment, said placental stem cells are obtained from a placenta of an infant born at 23 to 25 weeks of gestation, from a placenta of an infant born at 26 to 29 weeks of gestation, from a placenta of an infant born at 30 to 33 weeks of gestation, or from a placenta of an infant born at 34 to 37 weeks of gestation.

According to a further embodiment, said umbilical cord blood or said placental stem cells are not immunotyped prior to said use.

According to a further embodiment, said umbilical cord blood is prepared to be administered once after birth of the premature infant, a plurality of times after birth of the premature infant, within one hour after birth of the premature infant, within 12 hours after birth of the premature infant, within 24 hours after birth of the premature infant, or within one week after birth of the premature infant.

According to a further embodiment, said umbilical cord blood comprises 1 x 10⁵ to 1 x 10⁶ CD34⁺ cells per kilogram body weight of said premature infant.

According to a further embodiment, said placental stem cells and said umbilical cord blood together comprises 1 x 10⁵ to 1 x 10⁶ CD34⁺ cells per kilogram body weight of said premature infant.

According to a further embodiment, said umbilical cord blood is prepared to be administered by intravenous injection.

According to a further embodiment, said umbilical cord blood is prepared to be administered within 1 hour after birth, within 12 hours after birth, within 2 days after birth, or within 2 weeks after birth.

Within the boundaries of the present invention, reference to placental stem cells of the invention in the following description implies that such cells comprise cells that are CD34⁻, CD73⁺, CD105⁺, and CD200⁺. The cells may optionally be positive or negative for other cells markers.

In one aspect, the present invention provides umbilical cord blood comprising placental stem cells for use in a method of treating a disorder or condition of a premature infant, wherein the disorder or condition is caused by or associated with incomplete development of the brain, comprising administering to the premature infant a composition comprising umbilical cord blood, *e.g*., allogeneic cord blood. In specific embodiments, the method additionally comprises administering to the premature infant placental stem cells and/or blood additives. In embodiments in which placental stem cells and/or blood additives are administered, the umbilical cord blood can be autologous or allogeneic. In a more specific embodiment, said blood additive is erythropoietin, an iron supplement, a vitamin, or allogeneic red blood cells not obtained from cord blood. In a more specific embodiment, wherein said additive is allogeneic red blood cells, said cells are irradiated to a degree sufficient to reduce or prevent graft versus host disease. In a specific embodiment, said irradiation is with at least 2,500 cGy of radiation. In another more specific embodiment, wherein said additive is allogeneic red blood cells, said cells have been leukodepleted.

A premature infant is an infant who is born less than 37 weeks of gestation. In certain embodiments, the premature infant has undergone from 23 to 25 weeks of gestation at birth. In certain embodiments, the premature infant has under undergone from 26 to 29 weeks of gestation at birth. In certain embodiments, the premature infant has under undergone from 30 to 33 weeks of gestation at birth. In certain embodiments, the premature infant has under undergone from 34 to 37 weeks of gestation at birth.

In certain embodiments, the premature infant weighs 800 grams or more at birth. In certain embodiments, the premature infant weighs 500 grams to 800 grams at birth. In certain embodiments, the premature infant weighs less than 500 grams at birth.

The disorder or condition to be treated according to the present invention can be any disorder or condition known in the art to be caused by or associated with incomplete development of the brain. In certain embodiments, the disorder or condition is Respiratory Distress Syndrome (RDS) or Acute Respiratory Distress Syndrome (ARDS). In certain embodiments, the disorder or condition is anemia. In certain embodiments, the disorder or condition is intraventricular hemorrhage, necrotizing enterocolitis, retinopathy of prematurity, chronic lung disease (bronchopulmonary dysplasia), an infection, patent ductus arteriosus, apnea, low blood pressure, or hyperbilirubinemia. In certain embodiments, the disorder or condition is caused by incomplete development of an organ, such as lung, eye, immune system, brain, heart, liver or kidney.

Umbilical cord blood used in the present invention can be collected by any technique known in the art. In certain embodiments, umbilical cord blood is obtained from a cord blood bank. In certain embodiments, umbilical cord blood is collected from a post-partum mammalian placenta. In some embodiments, umbilical cord blood is obtained from a post-partum mammalian placenta of a full-term birth. In other embodiments, umbilical cord blood is obtained from a post-partum mammalian placenta of a premature birth. The umbilical cord blood can be allogeneic to the premature infant to be treated, if administered alone, or can be allogeneic or autologous, or a combination of both, if administered with placental stem cells or blood additives.

Placental stem cells used in the present invention can be isolated and processed by any method known in the art. In certain embodiments, placental stem cells are obtained from a placenta of a full-term birth. In certain embodiments, placental stem cells are obtained from a placenta of a premature birth. The placental stem cells useful in the methods of the invention can be allogeneic or autologous to the premature infant to be treated, or a combination of both.

In particular embodiments, placental stem cells are obtained from a placenta that has been exsanguinated and perfused to remove residual blood cells. The exsanguinated placenta can then be cultured for 2 to 24 hours, or more, under conditions appropriate to allow for the production of endogenous stem cells originating from the placenta.

Once obtained from a cultured placenta, the placental stem cells may be characterized by a number of methods, including but not limited to, immunochemistry to identify particular cell surface markers. Preferred stem cells to be used in accordance with the present invention may be identified by the presence of the following cell surface markers: CD34⁻, OCT-4⁺, CD73⁺, CD105⁺, CD200⁺ and/or HLA-G⁺. In certain embodiments, the placental stem cells comprise CD34⁺ cells. In certain embodiments, the placental stem cells comprise CD34⁻ cells. In certain embodiments, the placental stem cells comprise OCT-4⁺ cells. In certain embodiments, the placental stem cells comprise cells that are CD73⁺, CD105⁺ and CD200⁺. In certain embodiments, the placental stem cells comprise cells that are CD200⁺ or OCT-4⁺. In certain embodiments, said placental stem cells comprise cells that are CD200⁺ and OCT-4⁺. In certain embodiments, the placental stem cells comprise cells that are CD73⁺ and CD105⁺ and that facilitate the formation of one or more embryoid-like bodies in a population of placental cells comprising said cells when said population is cultured under conditions that allow the formation of an embryoid-like body. In certain embodiments, the placental stem cells comprise cells that are CD73⁺, CD105⁺ and CD200⁺. In certain embodiments, the placental stem cells comprise cells that are OCT-4⁺ and that facilitates the formation of one or more embryoid-like bodies in a population of placental cells comprising the stem cell when said population is cultured under conditions that allow formation of embryoid-like bodies.

The step of administering to the premature infant umbilical cord blood, or combinations of umbilical cord blood and placental stem cells, can be carried out according to the judgment of those of skill in the art, and can be performed, e.g., intravenously. Administration can be performed at various times after birth of the premature infant, but is generally preferred no more than about 2 weeks after birth. In various embodiments, administration is performed within one, two, five, ten, twelve, or twenty four hours, or one week, after birth of the premature infant. Administration can be performed once or a plurality of times after birth of the premature infant.

### 4. BRIEF DESCRIPTION OF THE DRAWINGS

FIG. 1 is a cross-sectional view of the cannulation of the vein and artery of a placenta to perfuse the placenta and then collect the perfusate.

FIGS. 2A-2E are schematics showing the collection, clamping, perfusion, collection and storage of a drained and perfused placenta.

FIG. 3 is a cross-sectional schematic of a perfused placenta in a device for use as a bioreactor.

### 5. DETAILED DESCRIPTION OF THE INVENTION

### 5.1 TREATMENT OF PREMATURE INFANTS

The present invention generally relates to umbilical cord blood comprising placental stem cells for use in methods of treating disorders and conditions of a premature infant caused by or associated with premature birth. The method comprises the step of administering to the premature infant umbilical cord blood comprising, placental stem cells.

Specifically, the present invention provides umbilical cord blood comprising placental stem cells for use in a method of treating a disorder or condition in a premature infant, wherein said disorder or condition is caused by or associated with incomplete development of the brain, wherein said placental stem cells comprise cells that are CD34⁻, CD73⁺, CD105⁺, and CD200⁺.

According to one embodiment, the umbilical cord further comprising a blood additive, wherein said blood additive is erythropoietin, an iron supplement, a vitamin, or red blood cells from a source other than cord blood.

According to another embodiment, said blood additive is erythropoietin.

According to a further embodiment, said erythropoietin is recombinant erythropoietin genetically engineered to increase serum half-life compared to native erythropoietin.

According to another embodiment, said blood additive is a vitamin.

According to a further embodiment, said vitamin is riboflavin (vitamin B2), pyridoxine (vitamin B6), folic acid, vitamin B 12, or vitamin E.

According to another embodiment, said blood additive is an iron supplement.

According to a further embodiment, said iron supplement is elemental iron.

According to another embodiment, said blood additive is red blood cells not obtained from cord blood.

According to a further embodiment, said red blood cells have been irradiated with at least 2500 cGy of radiation, or wherein said red blood cells have been leukodepleted.

According to a further embodiment, said premature infant has undergone from 23 to 25 weeks of gestation at birth, from 26 to 29 weeks of gestation at birth, from 30 to 33 weeks of gestation at birth, or from 34 to 37 weeks of gestation at birth.

According to a further embodiment, said premature infant weighs 800 grams or more at birth, 500 grams to 800 grams at birth, or less than 500 grams at birth.

According to a further embodiment, said umbilical cord blood is autologous to the premature infant.

According to a further embodiment, said placental stem cells are autologous to the premature infant.

According to a further embodiment, said umbilical cord blood is obtained from a post-partum mammalian placenta of a full-term birth.

According to a further embodiment, said umbilical cord blood is obtained from a post-partum mammalian placenta of a premature birth.

According to a further embodiment, said placenta is the placenta of said premature infant, the placenta of an infant born at 26 to 29 weeks of gestation, the placenta of an infant born at 30 to 33 weeks of gestation, or the placenta of an infant born at 34 to 37 weeks of gestation.

According to a further embodiment, said umbilical cord blood is obtained from a cord blood bank.

According to a further embodiment, said placental stem cells are stem cells isolated from placental perfusate prior to said use.

According to a further embodiment, said placental stem cells are stem cells contained within placental perfusate.

According to a further embodiment, said placental stem cells are obtained from a post-partum mammalian placenta of a full-term birth, from a post-partum mammalian placenta of a premature birth, or from a placenta of said premature infant.

According to a further embodiment, said placental stem cells are obtained from a placenta of an infant born at 23 to 25 weeks of gestation, from a placenta of an infant born at 26 to 29 weeks of gestation, from a placenta of an infant born at 30 to 33 weeks of gestation, or from a placenta of an infant born at 34 to 37 weeks of gestation.

According to a further embodiment, said umbilical cord blood or said placental stem cells are not immunotyped prior to said use.

According to a further embodiment, said umbilical cord blood is prepared to be administered once after birth of the premature infant, a plurality of times after birth of the premature infant, within one hour after birth of the premature infant, within 12 hours after birth of the premature infant, within 24 hours after birth of the premature infant, or within one week after birth of the premature infant.

According to a further embodiment, said umbilical cord blood comprises 1 x 10⁵ to 1 x 10⁶ CD34⁺ cells per kilogram body weight of said premature infant.

According to a further embodiment, said placental stem cells and said umbilical cord blood together comprises 1 x 10⁵ to 1 x 10⁶ CD34⁺ cells per kilogram body weight of said premature infant.

According to a further embodiment, said umbilical cord blood is prepared to be administered by intravenous injection.

According to a further embodiment, said umbilical cord blood is prepared to be administered within 1 hour after birth, within 12 hours after birth, within 2 days after birth, or within 2 weeks after birth.

In embodiments in which the cord blood is administered alone or with blood additives, the cord blood is allogeneic to the premature infant to be treated. In embodiments in which the cord blood is administered with placental stem cells and/or blood additives, the cord blood can be autologous or allogeneic to the recipient premature infant.

An infant is considered an extremely low birth weight (ELBW) infant if the infant is born weighing less than 1 kg (approximately 2.3 lbs). In certain embodiments, the premature infant weighs 800 grams or more at birth. In certain embodiments, the premature infant weighs at 500 grams to 800 grams at birth. In certain embodiments, the premature infant weighs less than 500 grams at birth.

As used herein, the terms "treat," "treating" and "treatment" refer to the cure, remediation, or the reduction or amelioration of the progression, severity, and/or duration, of a disorder or condition, in this case one caused by or related to premature birth, or any parameter or symptom of such a disorder or condition.

Treatment of a premature infant with umbilical cord blood, and optionally placental stem cells, may be considered efficacious if the premature infant survives, or if the disorder or condition caused by or associated with premature birth is measurably improved in any way as a result of the treatment. Such improvement may be shown by, e.g., one or more measurable indicators including, for example, detectable changes in a physiological condition or set of physiological conditions associated with a particular disease, disorder or condition (including, but not limited to, blood pressure, heart rate, respiratory rate, counts of various blood cell types, levels in the blood of certain proteins, carbohydrates, lipids or cytokines or modulation of expression of genetic markers associated with the disease, disorder or condition).

Treatment of a premature infant with umbilical cord blood, and optionally placental stem cells, is considered effective if any one of such indicators appears to respond to such treatment by changing to a value that is within, or closer to, a normal value for, e.g. full-term infants, than such indicator(s) would be expected to lie in the absence of administration of umbilical cord blood and/or placental stem cells. The normal value may be, a normal value or range of normal values that is known in the art for an indicator. For example, one or more metabolic or biochemical indicator displayed by a premature infant can be compared to the normal range for the indicator(s), wherein treatment is considered effective if the treatment results in the one or more metabolic or biochemical indicator more closely approaching, or falling within, a reference range for normal, full-term infants. Such indicator include, but are not limited to, levels of, or values for, 17 hydroxyprogesterone, 25-hydroxyvitamin D (25(OH)D), acetoacetate, acidity (pH), albumin, ammonia, amylase, ascorbic acid, bicarbonate, bilirubin, blood volume, calcium, carbon, dioxide partial pressure, carbon monoxide, CD4 cell count, ceruloplasmin, chloride, copper, creatine kinase (CK or CPK), creatine kinase isoenzymes, creatinine, erythrocyte sedimentation rate (ESR or Sed-Rate), globulin, glucose, hematocrit, hemoglobin, iron, iron-binding capacity, lactate (lactic acid) (arterial), lactic dehydrogenase, lipase, magnesium, mean corpuscular hemoglobin (MCH), mean corpuscular hemoglobin concentration (MCHC), mean corpuscular volume (MCV), osmolality, oxygen partial pressure, oxygen saturation (arterial), phosphatase, phosphorus, platelet count, potassium, protein (total), prothrombin (PTT), pyruvic acid, red blood cell count (RBC), sodium, thyroid-stimulating hormone (TSH), transaminase (alanine or aspartate), urea nitrogen (BUN) and BUN/creatinine ratio, uric acid, vitamin A, WBC (leukocyte count and white blood cell count), zinc, and the like.

The effectiveness of administration of cord blood, or cord blood and placental stem cells, can be assessed by behavioral tests. For example, improvement in neurodevelopment of a premature infant can be assessed, e.g., within the first 2-3 years of birth by one or more such tests as the Neonatal Neurobehavioral Examination, Alberta Infant Motor Scale, or Bayley Scale of infant Development (3d Edition), by comparing a score on such a test by the premature infant to a score, or range of scores, for normal and premature infants of different gestational ages, and determining that improvement takes place if the score is higher than the score is expected to be at the premature infant's gestational age. In a specific embodiment, the premature infant, receiving cord blood or a combination of cord blood and placental stem cells, is deemed to have improved if the score is higher than a score, or an average of scores, of premature infants of the same gestational age treated with only conventional treatments.

In one embodiment, a preterm infant is assessed shortly after birth (e.g., within the first week) by the Neonatal Neurobehavioral Examination (NNE) and given a score representing development of behavioral traits, primitive reflexes, and tone and motor patterns, wherein the maximum score is 81. The infant is re-assessed one or more times within two years of birth, preferably at 18 to 22 months. A significant improvement in the NNE score during this time indicates efficacy. In various embodiments, administration of umbilical cord blood and placental stem cells is effective if the score improves from average scores of, *e*.*g*., 37-42 week gestational age preterm infants (66.5) if the premature infant was born at 37-42 weeks gestational age; 34-36 week preterm infants (60.7) if the premature infant was born at 34-36 weeks gestational age, or infants born at 34 weeks or less gestational age (51.1) if the premature infant was born at 34 weeks or less gestational age, compared with premature infants not treated with cord blood or a combination of cord blood and placental stem cells. *See* Morgan, "Neonatal Neurobehavioral Examination. A New Instrument For Quantitative Analysis of Neonatal Neurological Status," Phys. Ther. 68(9): 1352-1358 (1988).

### 5.1.1 Disorders or Conditions Caused by or Associated with Premature Birth

The disorder or condition to be treated with the present invention can be any disorder or condition, caused by or associated with premature birth, that is known in the art. In certain embodiments, the disorder or condition is Respiratory Distress Syndrome

(RDS) or Acute Respiratory Distress Syndrome (ARDS). In certain embodiments, the disorder or condition is anemia. In certain embodiments, the disorder or condition is intraventricular hemorrhage, necrotizing enterocolitis, retinopathy of prematurity, chronic lung disease (bronchopulmonary dysplasia), an infection, patent ductus arteriosus, apnea, low blood pressure, or hyperbilirubinemia. In certain embodiments, the disorder or condition is caused by incomplete development of an organ, including but not limited to lung, eye, immune system, brain, heart, liver or kidney.

(Acute) Respiratory Distress Syndrome (ARDS/RDS) or Infant Respiratory Distress Syndrome (IRDS) (formerly called hyaline membrane disease) is a breathing disorder in which the air sacs (alveoli) in an infant's lungs do not stay open because of high surface tension resulting from insufficient production of surfactant. Respiratory distress syndrome affects 10% of all premature infants and only rarely affects those born at full-term. The disease is caused by a lack of lung surfactant, a chemical that normally appears in mature lungs. Surfactant keeps the air sacs from collapsing and allows them to inflate with air more easily. In respiratory distress syndrome, the air sacs collapse and prevent the child from breathing properly. Symptoms usually appear shortly after birth and become progressively more severe. Infants with RDS/ARDS usually need support with oxygen and a respirator or are treated with a surfactant drug.

Anemia is a disorder characterized by an insufficient number of erythrocytes or hemoglobins in the blood to carry adequate oxygen to the body. Premature infants may develop anemia for a number of reasons, including blood loss during delivery, lack of iron content and shorter half-life of red blood cells as compared to adults. Current treatment options include blood transfusion (from blood bank or directed donor blood obtained from family members), iron supplementation and preventing of blood loss. Although not intending to be bound by any particular theory, it is believed that umbilical cord blood can be a good source of blood for transfusion. Autologous infusion of umbilical cord blood has several advantages over blood from a blood bank or related donors: 1) umbilical cord blood is a good source of erythrocytes with moderate capacity to carry oxygen; 2) umbilical cord blood is readily available and requires minimal testing; and 3) the umbilical cord blood is a rich source of stem and progenitor cells capable of supporting further development of immature organs or organs damaged due to premature delivery.

Apnea is a disorder in which a premature infant temporarily stops breathing and is usually defined as cessation of breathing for 15 to 20 seconds. Apnea may occur in infants born before 34 weeks of pregnancy, increasing in frequency among the most prematurely born infants. It is thought to be caused by immaturity of the part of the brain that controls breathing. Infants with apnea are treated with drugs (*e.g*., aminophylline, caffeine, or doxapram), and/or are supported with continuous positive airway pressure or a ventilator.

Chronic lung disease (bronchopulmonary dysplasia) is a condition that develops in premature infants on mechanical ventilation and/or high oxygen levels for extended periods. Chronic lung disease is treated with oxygen, drugs and gradual weaning infants from the ventilator.

Hyperbilirubinemia, one of the most common problems encountered in newborns, is an abnormally high level of bilirubin in the blood. It is defined as a total serum bilirubin level greater than 5 mg/dL. Bilirubin above this level is neurotoxic/cytotoxic. It results from the deposition of unconjugated bilirubin pigment in the skin and mucus membranes. Mild hyperbilrubinemia does not require treatment. Higher bilirubin levels can be treated by phototherapy, in which the infant is placed under bilirubin lights.

Premature infants are at higher risk of developing infections than full-term babies, since their immune systems are immature. Serious infections seen in premature babies include sepsis, pneumonia and meningitis (infection of the membranes surrounding the brain). Currently, infections are treated with antibiotics or antiviral drugs.

Intraventricular hemorrhage is bleeding in the brain. It occurs when small blood vessels lying alongside the ventricles rupture. Intraventricular hemorrhage most frequently affects premature newborns Severe bleeding can cause brain damage, and can result in, *e.g*., learning disabilities and/or behavior problems.

Necrotizing enterocolitis is a condition in which the inner surface of the intestine becomes injured and inflamed; if severe, a portion of the intestine may die, leading to intestinal perforation and peritonitis. Necrotizing enteroclitis occurs mainly in premature infants. The cause for this disorder is unknown, but it is thought that a decreased blood flow to the bowel keeps the bowel from producing the normal protective mucus. Bacteria in the intestine may also be a cause. Necrotizing enterocolitis can lead to feeding problems, swelling in infants' belly and other complications. Necrotizing enterocolitis is treated with drugs and sometimes surgery.

Patent ductus arteriosus (PDA) is a condition where the ductus arteriosus, a blood vessel that allows blood to bypass the infant's lungs before birth, fails to close after birth. Patent ductus arteriosus is currently treated with drugs and surgery if necessary.

Retinopathy of prematurity is a disorder in which blood vessels in the back of the eye (retina) develop abnormally in premature infants; these blood vessels may bleed, and in the most severe cases, the retina may detach, leading to visual loss. It occurs mainly in infants born before the 32d week of pregnancy. The main risk factor for developing retinopathy of prematurity is extreme prematurity; high oxygen levels in the blood from the treatment of breathing problems may increase the risk. A bilateral retinopathy typically occurring in premature infants treated with high concentrations of oxygen, characterized by vascular dilatation, proliferation, and tortuosity, edema, and retinal detachment, with ultimate conversion of the retina into a fibrous mass that can be seen as a dense retrolental membrane. Typically, growth of the eye is arrested and may result in microphthalmia, and blindness may occur. Mild retinopathy of prematurity often heals spontaneously; infants with severe retinopathy are currently treated with a laser or cryotherapy, in which the peripheral portions of the retina are frozen.

In addition, the present invention also encompasses the treatment of disorders and conditions caused by incomplete development of certain organs, including but not limited to lung, eye, immune system, brain, heart, live and kidney. Currently, there are no treatment options for incomplete organ development.

### 5.1.2 Administration of Umbilical Cord Blood and Placental Stem Cells to Premature Infants

Umbilical cord blood, and placental stem cells, may be administered to a premature infant, in particular a premature infant having or exhibiting any disorder or condition associated with, or caused by, prematurity, in any pharmaceutically or medically acceptable manner, including by injection or transfusion. In certain embodiments, umbilical cord blood and placental stem cells are administered to a premature infant parenterally. The term "parenteral" as used herein includes subcutaneous injections, intravenous, intramuscular, intra-arterial injection, or infusion techniques. In preferred embodiments, umbilical cord blood, and optionally placental stem cells, are administered to a premature infant intravenously.

Umbilical cord blood or placental stem cells may be contained in any pharmaceutically-acceptable carrier. The umbilical cord blood or placental stem cells may be carried, stored, or transported in any pharmaceutically or medically acceptable container, for example, a blood bag, transfer bag, plastic tube, syringe, vial, or the like.

The step of administering umbilical cord blood, and optionally placental stem cells, to the premature infant can be carried out in any medically-acceptable manner. Administration can be performed at various times after birth of the premature infant. In various embodiments, for example, administration is performed within 1, 2, 3, 4, 5, 6, 7, 8, 9, 10, 11, 12, 15, 18, 21, or 24 hours after birth, or within 2, 3, 4, 5, or 6 days after birth, or within 1 or 2 weeks after birth.

Administration of cord blood, and optionally of placental stem cells, can be performed once or a plurality of times after birth of the premature infant. In certain embodiments, administration is performed once after birth of a premature infant. In certain embodiments, administration is performed a plurality of times after birth of a premature infant.

The amount or number of umbilical cord blood and placental stem cells administered to the premature infant depends on the source of umbilical cord blood and placental stem cells used, the severity or nature of disorders or conditions to be treated, as well as age, body weight and physical condition of the premature infant, *etc.* In certain embodiments, 0.01 to 0.1, 0.1 to 1, 1 to 10, 10 to 10², 10² to 10³, 10³ to 10⁴, 10⁴ to 10⁵, 10⁵ to 10⁶, 10⁶ to 10⁷, 10⁷ to 10⁸, or 10⁸ to 10⁹ umbilical cord blood cells, placental stem cells, or umbilical cord blood cells and placental stem cells per kilogram body weight of a premature infant are administered. In a specific embodiment, said umbilical cord blood cells are CD34⁺ cells. In another specific embodiment, said placental stem cells are CD34⁺ cells. Preferably, at least 10⁵ to 10⁷ CD34⁺ cells per kilogram body weight are administered. Such CD34⁺ cells can be from cord blood alone, or can be from cord blood and placenta. In various embodiments, at least 0.1, 1, 10, 10², 10³, 10⁴, 10⁵, 10⁶, 10⁷, 10⁸, or 10⁹ umbilical cord blood cells, placental stem cells, or umbilical cord blood cells and placental stem cells per kilogram body weight of a premature infant are administered. In various embodiments, at most 10⁴, 10⁵, 10⁶, 10⁷, 10⁸, or 10⁹ umbilical cord blood cells, placental stem cells, or umbilical cord blood cells and placental stem cells per kilogram body weight of a premature infant are administered. In specific embodiments of the above embodiments, the cord blood and/or placental stem cells are CD34⁺ cells.

The umbilical cord blood, and placental stem cells, is preferably delivered in a volume appropriate for the size of the premature infant. Typical blood volume of premature infants is 85-100 mL/kg body weight. Thus, the blood volume for premature infants ranges from approximately 40 mL to approximately 300 mL. In various embodiments, therefore, umbilical cord blood, and optionally placental stem cells, is administered in a total volume of 0.5 mL, 1.0 mL, 2 mL, 3 mL, 4 mL, 5 mL, 6 mL, 7 mL, 8 mL, 9 mL, 10 mL, 11 mL, 12 mL, 13 mL, 14 mL, 15 mL, 16 mL, 17 mL, 18 mL, 19 mL, or about 20 mL, or more. The administration of such volumes can be a single administration or in multiple administrations. Where the infant is particularly low birth weight (*e.g*., less than 1 kg), a desired volume of umbilical cord blood, or number of umbilical cord blood cells, and optionally placental stem cells, can be provided to the infant in a plurality of administrations. The time over which such volumes of cord blood or combinations of cord blood and placental stem cells can be administered can vary from, *e.g.,* 0.5 hours, 1 hour, 1.5 hours, 2 hours, 2.5 hours, 3 hours, 3.5 hours, 4 hours, or more.

Generally, small transfusions under 20 mL do not require a pump and may be pushed in via a syringe by, *e*.*g.*, an intermittent small bolus, taking into consideration the volume-tolerance of the infant. Larger-volume transfusions are preferably administered by an infusion device, within a period of two to four hours. If the transfusion interval is to exceed four hours, the blood component should be subdivided, and its second portion stored, *e.g*. in a blood bank, until needed.

### 5.1.3 Blood Additives

In certain embodiments, the cord blood administered to the premature infant comprises one or more additives. For example, such additives can include, *e.g*., erythropoietin, an iron supplement, a vitamin, or allogeneic red blood cells.

In a specific embodiment, the blood additive is erythropoietin. Erythropoietin can be administered in any medically-acceptable form. The erythropoietin can be native erythropoietin purified from a biological source, or an engineered erythropoietin such as EPOGEN®, ARANESP® or PROCRIT®. The erythropoietin can have the sequence of native human erythropoietin, or can be an erythropoietin engineered to increase serum half-life. Typical dosages for erythropoietin range from 500-1500 U/kg/week.

In another specific embodiment, the blood additive is iron or an iron supplement. The iron or iron supplement can be in any metabolically available and medically acceptable form, but is preferably elemental iron. Typical dosages for preterm infants are from 4.0 mg/kg/day to 4.5 mg/kg/day, where the preterm infant weighs 1500 grams or less, and 2 mg/kg/day where the infant weighs 1500 grams to 2500 grams.

In another specific embodiment, the blood additive is, or comprises, one or more vitamins. In preferred embodiments, the one or more vitamins are those vitamins necessary for blood development, including, but not limited to, riboflavin (vitamin B2), pyridoxine (vitamin B6), folic acid, vitamin B12, and/or vitamin E. In one embodiment, about 25 IU of vitamin E is administered to the premature infant. Such vitamins can be administered to the premature infant at approximately the same dosage, per kilogram, as established for adults. In various embodiments, a single administration of cord blood and, optionally, placental stem cells, includes 150 µg riboflavin; 100 µg pyridoxine; 25 µg folic acid; 0.4 µg vitamin B12; and/or 3.6 IU vitamin E. The blood additive can also be one or more other vitamins, *e.g*., vitamin A (*e.g.,* 460 IU per administration); vitamin D (*e.g*., 70 IU per administration); vitamin K (*e.g*., 11 µg per administration); thiamin (vitamin B1, *e.g*., 220 µg per administration); niacin (*e.g*., 1950 µg per administration); pantothenic acid (*e.g.,* 800 µg per administration); biotin (*e.g.,* 9 µg per administration); vitamin C (ascorbic acid, *e.g.,* 15 mg per administration); inositol (*e.g.,* 6 mg per administration); and/or linoleic acid (*e.g*., 750 mg per administration). The blood additive can be, or can comprise, any combination of the foregoing vitamins.

In another specific embodiment, the blood additive is allogeneic red blood cells from a source other than cord blood, *e.g.,* red blood cells from peripheral blood. In a preferred embodiment, where the donor is a first- or second-degree relative, the red blood cells are irradiated to a degree sufficient to reduce or prevent graft versus host disease. For example, in one embodiment, red blood cells are irradiated with at least 2,500 cGy prior to administration. Such irradiated red blood cells are preferably administered within 24 hours of irradiation. In other embodiments, the red blood cells administered to the premature infant with the cord blood are from an unrelated donor. Preferably, such red blood cells are from a single unrelated donor. In a preferred embodiment, the red blood cells are collected from the unrelated donor using a multipack blood collection system, which enables multiple administrations to the premature infant from the same donor, reducing immunological complications. In another more specific embodiment, wherein said additive is allogeneic red blood cells, said cells have been leukodepleted, *e.g.,* using a leukodepletion filter. *See, e.g.,* U.S. Patent No. 5,728,306. In all embodiments in which red blood cells are used as a blood additive, it is preferred that the red blood cells have been stored for no more than five days prior to administration. In a preferred embodiment, the red blood cells comprise adenine-saline (AS-3) anticoagulant.

In another preferred embodiment, the red blood cells are from units of donated blood that have been tested for the absence of at least the following pathogens, or absence of antibodies to the following pathogens: human immunodeficiency virus (HIV)-I, HIV-II, hepatitis C virus, human T-lymphotrophic virus (HTLV)-I and HTLV-II, hepatitis B virus (HBV, as evidenced, *e.g.,* by absence of hepatitis B virus surface antigen (HBsAg)), cytomegalovirus, and syphilis.

### 5.1.4 Combination Therapy

In certain embodiments of the umbilical cord blood comprising placental stem cells for use in methods provided herein, umbilical cord blood, and placental stem cells, are used as a first therapy in combination with one or more second therapies in the treatment of a disorder or condition of a premature infant. Such second therapies include, but are not limited to, surgery, hormone therapy, immunotherapy, phototherapy or treatment with certain drugs.

The use of the term "combination therapy" does not limit the order in which treatments are administered to a premature infant in the methods provided. For example, the agents of the combination therapy can be administered concurrently, sequentially in any order or cyclically to a premature infant. In some embodiments, two components of a combination therapy are administered concurrently to a premature infant.

Components of combination therapy can be administered to a premature infant in the same pharmaceutical composition. Alternatively, components of combination therapies can be administered to a premature infant in separate pharmaceutical compositions, and these separate compositions may be administered by the same or by different routes of administration, including, for example, oral, parenteral (*e.g.,* ocular, nasal, dermal, muscular or peritoneal route(s), and the like), or topical, *etc.*

Particular second therapies are carried out according to the therapies' respective standard or art-recognized doses and dosing schedules.

In certain embodiments, a second therapeutic agent, and/or optional third therapeutic agent, is selected for its additive effects with umbilical cord blood and placental stem cells in the treatment of a disorder or condition of a premature infant.

In certain embodiments, a second therapeutic agent, and/or optional third therapeutic agent, is selected for its synergistic effects with umbilical cord blood and placental stem cells in the treatment of a disorder or condition of a premature infant.

Exemplary therapies that can be used in combination with administration of umbilical cord blood, and optionally placental stem cells, include control of environmental temperature; support with oxygen; a respirator or a ventilator; peripheral blood transfusion; iron supplementation; intravenous feeding; phototherapy; surgery; agents for the treatment of apnea (*e.g*., aminophylline, caffeine or doxapram, and the like); agents for the treatment of ARDS or RDS (*e.g.,* a surfactant drug such as, *e.g.,* CUROSURF® (Poractant Alfa; Douglas Pharmaceuticals)); antibiotics or antiviral drugs, anti-inflammatory agents (*e.g*., steroidal anti-inflammatory compounds, non-steroidal anti-inflammatory (NSAID) compounds), nitric oxide; antihistamines, immune suppressants, immunomodulatory compound (*e.g*., a TNF-α inhibitor); laser treatment (to treat, *e.g.,* retinopathy of prematurity); *etc.* The treatment of premature infants is well-known in the art, and persons of skill in the art are able to select particular therapies, suitable for use in combination with administration of cord blood, on a case-by-case basis.

### 5.2 UMBILICAL CORD BLOOD

Umbilical cord blood may be collected in any medically or pharmaceutically-acceptable manner. Various methods for the collection of cord blood have been described. *See, e.g.,* U.S. Pat. No. 6,102,871; U.S. Pat. No. 6,179,819 B1; and U.S. Pat. No. 7,147,626.

The conventional technique for the collection of cord blood is based on the use of a needle or cannula, which is used with the aid of gravity to drain the cord blood from the placenta. *See e.g.,* U.S. Pat. Nos. 5,192,553; 5,004,681; 5,372,581, and 5,415,665. Usually the needle or cannula is placed in the umbilical vein and the placenta is gently massaged to aid in draining the cord blood from the placenta. Cord blood may be collected into, for example, blood bags, transfer bags, or sterile plastic tubes.

In some embodiments, umbilical cord blood is obtained from a commercial cord blood bank (*e.g.,* LifeBankUSA, *etc*.). In another embodiments, umbilical cord blood is collected from a post-partum mammalian placenta and used immediately (*e.g*., within 1, 2, 3, 4, 5, 6, 7 , 8, 9, 10, 11 or 12 hours of collection). In other embodiments, the cord blood used to treat a premature infant is cord blood that has been cryopreserved. Umbilical cord blood can be collected from a single placenta or from a plurality of placentas.

Umbilical cord blood that is administered to a premature infant can be autologous or heterologous. In particular embodiments, umbilical cord blood is obtained from the placenta of the premature infant to be treated. In certain embodiments, umbilical cord blood is obtained from a post-partum mammalian placenta of a full-term birth. In other embodiments, umbilical cord blood is obtained from a post-partum mammalian placenta of a premature birth, *e.g*., the placenta of the premature infant. In some embodiments, the placenta is the placenta of an infant born at 23 to 25 weeks of gestation. In some embodiments, embodiments, the placenta is the placenta of an infant born at 26 to 29 weeks of gestation. In some embodiments, embodiments, the placenta is the placenta of an infant born at 30 to 33 weeks of gestation. In some embodiments, embodiments, the placenta is the placenta of an infant born at 34 to 37 weeks of gestation.

Cord blood or stem cells derived therefrom may be stored as collected from a single individual (*i*.*e.*, as a single unit) for administration, or may be pooled with other units. Where umbilical cord blood is pooled from a plurality of placentas, the pooled cord blood can comprise umbilical cord blood from full-term births only, cord blood from a combination of full-term births, or cord blood from premature births only. For example, cord blood from the placenta of the premature infant can be combined with, *e.g*., cord blood from other premature infants, cord blood from full-term births only, or a combination of cord blood from both premature and full-term placentas. Cord blood, including autologous or allogeneic cord blood, can also be combined with peripheral blood. Cord blood from premature births is preferable, as such cord blood comprises relatively high numbers of CD34⁺ stem cells per unit volume, compared to cord blood from full-term births.

In some embodiments, the total nucleated cells present in the cord blood comprises at least 5%, 10%, 15%, 20%, or more of CD38⁺CD45⁺ cells. In additional embodiments, the total nucleated cells present in the cord blood comprises at least 25%, 30%, 40%, 50%, or more of CD38⁻CD45⁻ cells. In some embodiments, the cord blood is prepared from preterm placenta. In other embodiments, the cord blood is prepared from full term placenta.

### 5.3 PLACENTAL STEM CELLS

As used herein, the term "placental stem cell" refers to a tissue culture plastic-adherent stem cell (*e.g.,* a multipotent cell) that is obtained from or derived from a mammalian placenta, or a portion thereof (*e.g*., amnion, chorion, and the like) regardless of morphology, cell surface markers, *etc.* The phrase encompasses a stem cell obtained directly from a placenta, *e.g*., as part of a population of placental cells in placental perfusate or digested placental tissue (digestate), or a stem cell that is part of a population of placental cells that has been expanded and/or passaged one or more times. The term does not, however, encompass stem cells derived solely from another tissue, *e.g.,* placental blood or umbilical cord blood. The placenta comprises stem cell populations having, and distinguishable from each other by, for example, distinct sets of markers. Placental stem cells, and methods of obtaining the same, are described in detail in U.S. Pat. No. 7,045,148; 7,255,879; and in U.S. Patent Application Publication No. 2007/0275362, filed December 26, 2006.

Placental stem cells can be recovered following successful birth and placental expulsion, resulting in the recovery of as many as one billion nucleated cells, which yield 50-100 million multipotent and pluripotent stem cells.

Placental stem cells useful in the methods and compositions of the invention include, for example, pluripotent cells, multipotent cells, committed progenitor cells, hematopoietic progenitor cells, and mesenchymal-like stem cells from placenta. In one embodiment, the placental stem cells are contained within, or are derived from, placental perfusate. In other embodiments, placental stem cells are contained within, or are derived from, placental tissue that has been digested with one or more tissue-digesting enzymes (*e.g*., collagenase, hyaluronidase, and the like).

Placenta-derived stem cells used in the methods of the invention can be derived from a single placenta, or from a plurality of placentas.

In certain embodiments, placental stem cells are obtained from a placental of a full-term birth. In certain embodiments, the placental stem cells are obtained from a placental of a premature birth. In some embodiments, embodiments, the placenta is the placenta of an infant born at 23 to 25 weeks of gestation. In some embodiments, embodiments, the placenta is the placenta of an infant born at 26 to 29 weeks of gestation. In some embodiments, embodiments, the placenta is the placenta of an infant born at 30 to 33 weeks of gestation. In some embodiments, embodiments, the placenta is the placenta of an infant born at 34 to 37 weeks of gestation.

Placental stem cells can be autologous or allogeneic to the particular premature infant to be treated. In particular embodiments, placental stem cells are obtained from the premature infant to be treated.

Placental stem cells used in the methods of the invention can be obtained by any method. Placental stem cells can be obtained by, for example, perfusion, as disclosed in U.S. Pat. No. 7,045,148.

Such perfusion can be perfusion by the pan method, wherein perfusion liquid is forced through the placental vasculature and perfusion fluid that exudes from the placenta, typically the maternal side, is collected in a pan containing the placenta. Perfusion can also be a closed-circuit perfusion, wherein perfusion fluid is passed through, and collected from, only the fetal vasculature of the placenta. In a specific embodiment, such perfusion can be continuous, that is, perfusion fluid that has been passed through the placenta, and which comprises a plurality of placental cells, is passed through a second time, or a plurality of times, prior to isolation of placental cells.

Placenta-derived stem cells may also be obtained by physical or enzymatic disruption of the placenta using, *e.g*., proteases and/or other tissue-disruptive enzymes to disrupt the multicellular structure of the placenta. Such proteases may include neutral proteases or metalloproteases, *e.g*., collagenase, dispase, trypsin, elastase, and the like. Placental stem cells may also be obtained by physical disruption of the placenta using, *e.g*., mucolytic enzymes, for example, hyaluronidase.

The isolated perfused placenta of the invention provides a source of large quantities of stem cells enriched for CD34⁺ stem cells, *e.g*., CD34⁺CD38⁻ stem cells, and CD34⁻ stem cells, *e*.*g*., CD34⁻CD38⁺ stem cells. The first collection of blood from the placenta is referred to as cord blood, which contains predominantly CD34⁺CD38⁺ hematopoietic progenitor cells. Within the first twenty-four hours of post-partum perfusion, CD34⁺CD38⁻ hematopoietic progenitor cells may be isolated from the placenta, along with CD34⁻CD38⁺ cells. After about twenty-four hours of perfusion, CD34-CD38- cells can be isolated from the placenta along with the aforementioned cells. An isolated placenta that has been perfused for twenty-four hours or more provides a source of large quantities of stem cells enriched for CD34⁻CD38⁻ stem cells.

At least one class of human placental stem cells has characteristics of embryonic stem or germ cells. For example, stem cells of this class are SSEA3⁻ (stage-specific embryonic antigen 3), SSEA4⁻, OCT-4⁺ (a stem cell transcription factor) and/or ABC-p⁺ (ATP-binding cassette (ABC) transporter protein), a marker profile exhibited by pluripotent stem cells that have not yet undergone differentiation. Thus, in one embodiment, placental stem cells useful in the methods of the invention are SSEA3⁻, SSEA4⁻, OCT-4⁺ and/or ABC-p⁺. In another embodiment, the embryonic-like stem cells of the invention are OCT-4⁺ and ABC-p⁺. In another embodiment, the human placental stem cells do not express MHC Class 2 antigens.

Umbilical cord blood comprising placental stem cells for use in the methods of the invention are CD10⁺, CD38⁻, CD29⁺, CD34⁻, CD44⁺, CD45⁻, CD54⁺, CD90⁺, SH2⁺, SH3⁺, SH4⁺, SSEA3⁻, SSEA4⁻, OCT-4⁺, and/or ABC-p.

Preferred umbilical cord blood comprising placental stem cells for use in the method of the present invention are CD34⁻, OCT-4⁺, CD73⁺, CD105⁺, CD200⁺ and HLA-G⁺. In certain embodiments, the placental stem cells comprise CD34- cells. In other embodiments, the placental stem cells comprise OCT-4⁺ cells. In other embodiments, the placental stem cells comprise cells that are CD73⁺, CD105⁺ and CD200⁺. In other embodiments, the placental stem cells comprise cells that are CD200⁺ or HLA-G⁺. In other embodiments, said placental stem cells comprise cells that are CD200⁺ and OCT-4⁺. In other embodiments, the placental stem cells comprise cells that are CD73⁺ and CD105⁺ and that facilitate the formation of one or more embryoid-like bodies in a population of placental cells comprising said cells when said population is cultured under conditions that allow the formation of an embryoid-like body. In other embodiments, the placental stem cells comprise cells that are CD73⁺, CD105⁺ and HLA-G⁺. In other embodiments, the placental stem cells comprise cells that are OCT-4⁺ and that facilitate the formation of one or more embryoid-like bodies in a population of placental cells comprising the stem cell when said population is cultured under conditions that allow formation of embryoid-like bodies.

Thus, in one embodiment, the invention provides methods of treating a premature infant comprising administering to the premature infant cord blood and placental stem cells. In a specific embodiment, said stem cells are CD200⁺ or HLA-G⁺. In a specific embodiment, the stem cells are also CD200⁺ and HLA-G⁺. In a more specific embodiment, said stem cells are also CD73⁺ and CD105⁺. In another more specific embodiment, said stem cells are also CD34⁻, CD38⁻ or CD45⁻. In another more specific embodiment, said stem cells are also CD34⁻, CD38⁻ and CD45⁻. In another more specific embodiment, said stem cells are also CD34⁻, CD38⁻, CD45⁻, CD73⁺ and CD105⁺. In another specific embodiment, said CD200⁺ or HLA-G⁺ stem cells facilitate the formation of embryoid-like bodies in a population of placenta-derived cells comprising the stem cells, under conditions that allow the formation of embryoid-like bodies.

In another specific embodiment, said placental stem cells are CD73⁺, CD105⁺ and CD200⁺. In a more specific embodiment, said stem cells are also HLA-G⁺. In another more specific embodiment, said stem cells are also CD34⁻, CD38⁻ or CD45⁻. In another more specific embodiment, said stem cells are also CD34⁻, CD38⁻ and CD45⁻. In a more specific embodiment, said stem cells are also CD34⁻, CD38⁻, CD45⁻, and HLA-G⁺. In another more specific embodiment, the CD73⁺, CD105⁺, and CD200⁺ stem cells facilitate the formation of one or more embryoid-like bodies in a population of placenta-derived cells comprising the stem cell, when the population is cultured under conditions that allow the formation of embryoid-like bodies.

In another specific embodiment, said placental stem cells are CD200⁺ and OCT-4⁺. In a more specific embodiment, the stem cells are also CD73⁺ and CD105⁺. In another more specific embodiment, said cells are also HLA-G⁺. In another specific embodiment, said stem cell is CD34⁻, CD38⁻ or CD45⁻. In another specific embodiment, said stem cell is CD34⁻, CD38⁻ and CD45⁻. In a more specific embodiment, said stem cell is CD34⁻, CD38⁻, CD45⁻, CD73⁺, CD105⁺ and HLA-G⁺. In another specific embodiment, the placental stem cells facilitate the production of one or more embryoid-like bodies by a population of placenta-derived cells that comprises the stem cells, when the population is cultured under conditions that allow the formation of embryoid-like bodies.

In another specific embodiment, said placental stem cells are CD73⁺, CD105⁺ and HLA-G⁺. In a more specific embodiment, said stem cells are CD34⁻, CD38⁻ or CD45⁻. In a more specific embodiment, said stem cells are CD34⁻, CD38⁻ and CD45⁻. In another more specific embodiment, said CD73⁺, CD105⁺ and HLA-G⁺ stem cells are OCT-4⁺. In another more specific embodiment, said stem cell is CD200⁺. In a more specific embodiment, said stem cell is CD34⁻, CD38⁻, CD45⁻, OCT-4⁺ and CD200⁺. In another more specific embodiment, said placental stem cell facilitates the formation of embryoid-like bodies in a population of placental cells comprising said stem cell, when the population is cultured under conditions that allow the formation of embryoid-like bodies.

In another specific embodiment, the placental stem cells can be one or more of SSEA3⁻, SSEA4⁻, OCT-4⁺ and ABC-p⁺. In another embodiment, the placental stem cells are OCT-4⁺ and ABC-p⁺. In one embodiment, the human placental stem cells do not express MHC Class II antigens. In other embodiments, the placental stem cells are one or more of CD10⁺, CD38⁻, CD29⁺, CD34⁻, CD44⁺, CD45⁻, CD54⁺, CD90⁺, SH2⁺, SH3⁺, SH4⁺, SSEA3⁻, SSEA4⁻, OCT-4⁺, and/or ABC-p⁺.

In another embodiment, the placental stem cells are homogenous, and sterile. In another specific embodiment, the placental stem cells are present in a pharmaceutical grade solution suitable for administration to a human.

Markers, such as cell surface markers, can be routinely determined according to methods well known in the art, *e.g.* by flow cytometry or fluorescence-activated cell sorting (FACS) analysis by washing and staining with an anti-cell surface marker antibody labeled with an appropriate fluorophore. For example, to determine the presence of CD34 or CD38, cells may be washed in PBS and then double-stained with anti-CD34 phycoerythrin and anti-CD38 fluorescein isothiocyanate (Becton Dickinson, Mountain View, CA). The cells would then be analyzed using a standard flow cytometer. Alternatively, intra-cellular markers can also be examined via standard methodology.

### 5.4 COLLECTION AND CHARACTERIZATION OF PLACENTAL

### STEM CELLS

Placental stem cells can be collected and isolated by any technique known to those of skill in the art. In preferred embodiments, placental stem cells are isolated and collected as described in U.S. Patent No. 7,045,148, or in U.S. Patent Application Publication No. 2007/0275362, filed December 26, 2006. Methods of collecting placental stem cells are described below.

### 5.4.1 Isolating Placental Stem Cells By Perfusion

### 5.4.1.1 Pretreatment of Placenta

The collection of placental stem cells starts with collection of a placenta, *e.g*., a human placenta. In certain embodiments, a human placenta is recovered shortly after its expulsion after birth and, in certain embodiments, the cord blood in the placenta is recovered. In specific embodiments, the placenta is subjected to a conventional cord blood recovery process as an adjunct to treatment of the premature infant, *e.g*., recovery of cord blood in response to the particular premature infant's need. Cord blood may also be obtained from a commercial cord blood banking service, *e.g*., LifeBankUSA, Cedar Knolls, N.J.

Typically, cord blood collection proceeds as follows. Postpartum (after either Caesarian delivery or natural birth), the placenta is exsanguinated, *e.g*., drained of cord blood. Prior to cord blood collection, the placenta may be stored under sterile conditions and at a temperature of, *e.g*., 5°C to 25°C, or at about room temperature. In certain embodiments, the proximal umbilical cord is clamped, preferably within 4-5 cm (centimeter) of the insertion into the placental disc prior to cord blood recovery. In other embodiments, the proximal umbilical cord is clamped after cord blood recovery but prior to further processing of the placenta. Conventional techniques for the collection of cord blood may be used. Typically a needle or cannula is used, with the aid of gravity, to drain cord blood from (*i.e.,* exsanguinate) the placenta (*see, e.g.,* Boyse et al., U.S. Pat. No. 5,192,553; Boyse et al., U.S. Pat. No. 5,004,681; Anderson, U.S. Pat. No. 5,372,581; Hessel et al., U.S. Pat. No. 5,415,665). The needle or cannula is usually placed in the umbilical vein and the placenta is gently massaged to aid in draining cord blood from the placenta.

The placenta may then be stored for a period of 1 hour to 72 hours, preferably 4 to 24 hours, prior to perfusing the placenta to remove any residual cord blood.

After cord blood collection, the placenta is preferably stored in an anticoagulant solution at a temperature of 5°C to 25°C, *e.g.,* at about room temperature. Suitable anticoagulant solutions are well known in the art. For example, a solution of heparin or warfarin sodium can be used. In a preferred embodiment, the anticoagulant solution comprises a solution of heparin (1% w/w in 1:1000 solution). The drained placenta is preferably stored for no more than 36 hours before placental stem cells are collected, as described below.

Typically, a placenta is transported from the delivery or birthing room to another location, *e.g*., a laboratory, for recovery of cord blood and/or drainage and stem cell collection by, *e.g*., perfusion or enzymatic digestion of placental tissue. The placenta is preferably transported in a sterile, thermally insulated transport device

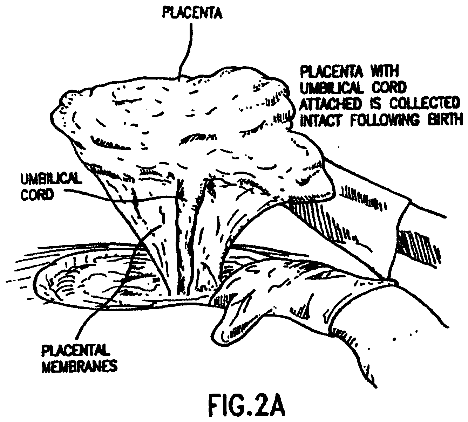

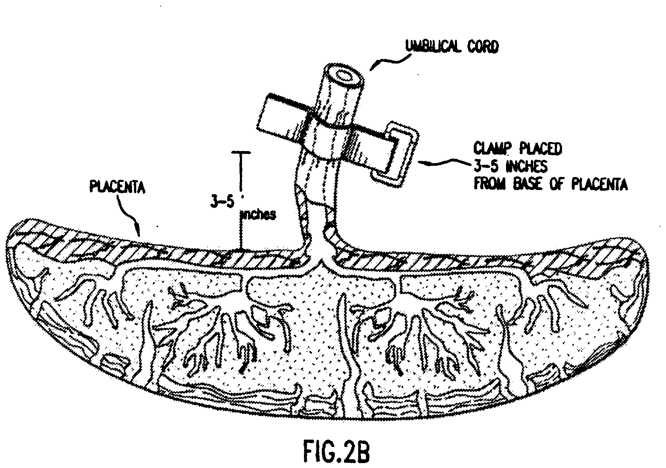

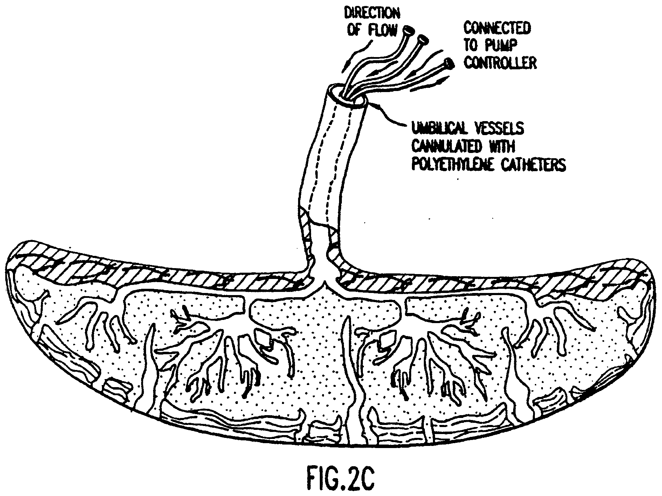

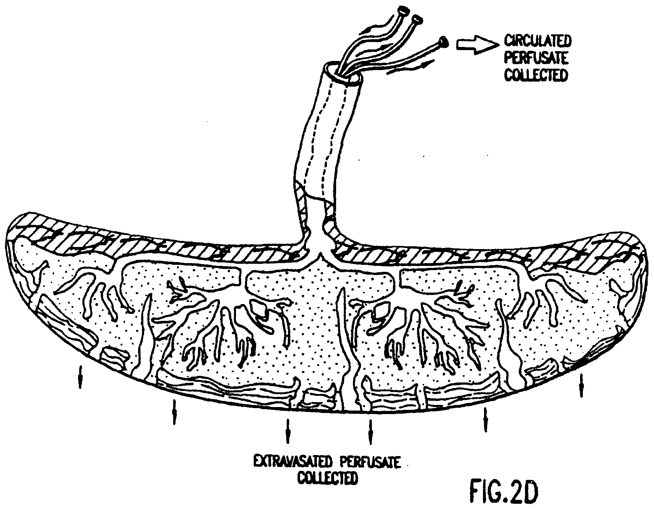

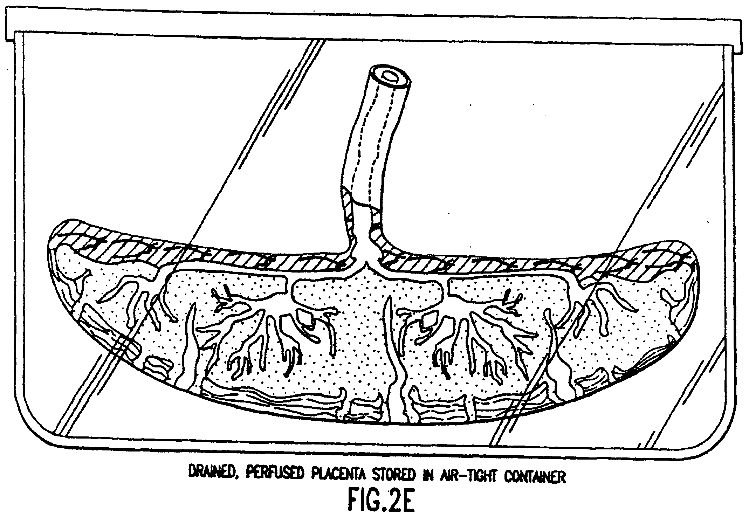

(maintaining the temperature of the placenta between, *e.g*., 20°C and 28°C), for example, by placing the placenta, with clamped proximal umbilical cord, in a sterile zip-lock plastic bag, which is then placed in an insulated container, as shown in FIGS. 2a-e.

In a preferred embodiment, the placenta is recovered from a patient by informed consent and a complete medical history of the patient prior to, during and after pregnancy is also taken and is associated with the placenta. These medical records can be used to coordinate subsequent use of the placenta or the stem cells harvested therefrom. For example, such human placental stem cells can then easily be used for personalized medicine for the premature infant to be treated.

### 5.4.1.2 Exsanguination of Placenta and Removal of Residual Cells

After cord blood recovery, placental stem cells are recovered from the placenta by, *e.g*., perfusion. In one aspect, the exsanguinated placenta is perfused with a suitable aqueous perfusion fluid to remove residual cord blood. The perfusion solution can be any aqueous isotonic fluid in which an anticoagulant (*e.g*., heparin, warfarin sodium) is preferably dissolved. Such aqueous isotonic fluids for perfusion are well known in the art, and include, *e.g.,* nutrient media, saline solutions, *e.g.,* phosphate buffered saline or, preferably, a 0.9 N sodium chloride solution. The perfusion fluid preferably comprises the anticoagulant at a concentration that is sufficient to prevent the formation of clots of any residual cord blood. In a specific embodiment, a concentration of from 1 to 100 units of heparin is employed, preferably a concentration of 1 to 10 units of heparin per ml is employed. In one embodiment, apoptosis inhibitors, such as free radical scavengers, in particular oxygen free radical scavengers, can be used during and immediately after exsanguination and then these agents can be washed from the placenta. In accordance with this embodiment of the invention, the isolated placenta may be stored under hypothermic conditions in order to prevent or inhibit apoptosis.

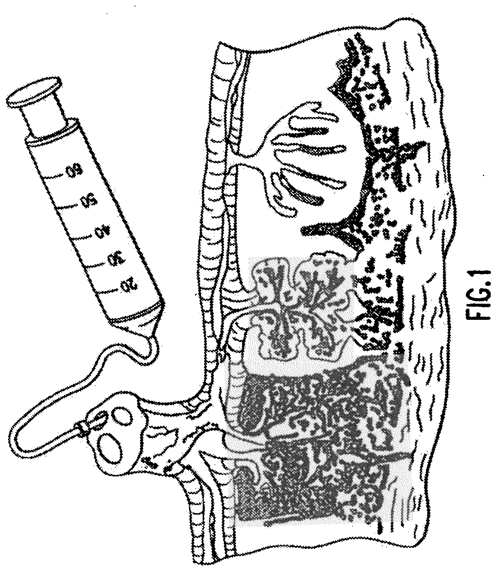

Preferably, prior to collection of placental stem cells, the placenta is flushed with, *e.g.,* 10-100 mL of perfusion fluid to remove substantially all remaining cord blood. Typically such flushing is performed by passage of the perfusion fluid through either or both of the umbilical artery and umbilical vein, using a gravity flow into the placenta. The placenta is preferably oriented (*e.g*., suspended) in such a manner that the umbilical artery and umbilical vein are located at the highest point of the placenta. In a preferred embodiment, the umbilical artery and the umbilical vein are connected simultaneously, as shown in FIG. 1, to a pipette that is connected via a flexible connector to a reservoir of the perfusion fluid. The perfusion fluid is passed into the umbilical vein and artery and collected in a suitable open vessel from the surface of the placenta that was attached to the uterus of the mother during gestation. The perfusion fluid may also be introduced through the umbilical cord opening and allowed to flow or percolate out of openings in the wall of the placenta which interfaced with the maternal uterine wall.

In a preferred embodiment, the proximal umbilical cord is clamped during perfusion, and more preferably, is clamped within 4-5 cm (centimeter) of the cord's insertion into the placental disc.

In one embodiment, a sufficient amount of perfusion fluid is used that will result in removal of all residual cord blood and subsequent collection or recovery of placental cells, including but not limited to embryonic-like stem cells and progenitor cells, that remain in the placenta after removal of the cord blood.

It has been observed that when perfusion fluid is first collected from a placenta during flushing, the fluid is colored with residual red blood cells of the cord blood. The perfusion fluid tends to become clearer as perfusion proceeds and the residual cord blood cells are washed out of the placenta. Generally from 30 to 100 ml (milliliter) of perfusion fluid is adequate to exsanguinate the placenta and to recover an initial population of embryonic-like cells from the placenta, but more or less perfusion fluid may be used depending on the observed results.

### 5.4.1.3 Culturing The Placenta

In preferred embodiments, the placenta is cultured or cultivated under aseptic conditions in a container or other suitable vessel, and perfused with perfusate solution (*e.g.,* a normal saline solution such as phosphate buffered saline ("PBS"), or, preferably, a 0.9 N saline solution) with or without an anticoagulant (*e.g*., heparin, warfarin sodium, coumarin, bishydroxycoumarin), and/or with or without an antimicrobial agent (*e.g*., β-mercaptoethanol (0.1 mM); antibiotics such as streptomycin (*e.g*., at 40-100 µg/ml), penicillin (*e.g*., at 40 U/ml), amphotericin B (*e.g.,* at 0.5 µg/ml), or the like. Various media may be used as perfusion fluid for culturing or cultivating the placenta, such as DMEM, Ham's F-12, M199, RPMI, Fisher's, Iscove's, McCoy's and combinations thereof, supplemented with fetal bovine serum (FBS), whole human serum (WHS), or human umbilical cord serum collected at the time of delivery of the placenta. The same perfusion fluid used to exsanguinate the placenta of residual cord blood may be used to culture or cultivate the placenta, without the addition of anticoagulant agents.

The effluent perfusate comprises both circulated perfusate, which has flowed through the placental circulation, and extravasated perfusate, which exudes from or passes through the walls of the blood vessels into the surrounding tissues of the placenta. The effluent perfusate, or circulated perfusate, or, preferably, both the circulated and extravasated perfusates are collected, preferably in a sterile receptacle. Alterations in the conditions in which the placenta is maintained and the nature of the perfusate can be made to modulate the volume and composition of the effluent perfusate.

Cell types are then isolated from the collected perfusate by employing techniques known by those skilled in the art, such as for example, but not limited to density gradient centrifugation, magnetic cell separation, cell sorting by FACS, affinity cell separation or differential adhesion techniques.

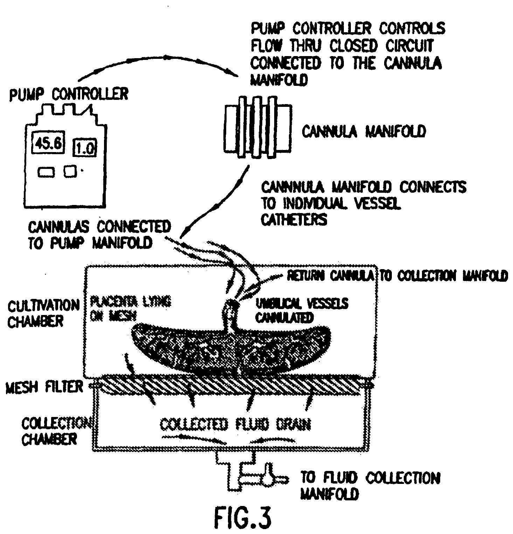

In one embodiment, a placenta is placed in a sterile basin and washed with 500 ml of phosphate-buffered normal saline. The wash fluid is then discarded. The umbilical vein is then cannulated with a cannula, *e.g*., a TEFLON® or plastic cannula, that is connected to a sterile connection apparatus, such as sterile tubing. The sterile connection apparatus is connected to a perfusion manifold, as shown in FIG. 3. The container containing the placenta is then covered and the placenta is maintained at room temperature (20-25°C) for a desired period of time, preferably from 2 to 24 hours, and preferably, no longer than 48 hours. The placenta may be perfused continually, with equal volumes of perfusate introduced and effluent perfusate removed or collected. Alternatively, the placenta may be perfused periodically, *e.g*., at every 2 hours; at 4, 8, 12, and 24 hours; or at other intervals during culturing, with a volume of perfusate, *e.g*., 100 ml of perfusate (sterile normal saline supplemented with or without 1000 u/l heparin and/or EDTA and/or CPDA (creatine phosphate dextrose)). In the case of periodic perfusion, preferably equal volumes of perfusate are introduced and removed from the culture environment of the placenta, so that a stable volume of perfusate bathes the placenta at all times.

The effluent perfusate that escapes the placenta, e.g., at the opposite surface of the placenta, is collected and processed to isolate embryonic-like stem cells, progenitor cells or other cells of interest.

The number and type of cells propagated may easily be monitored by measuring changes in morphology and cell surface markers using standard cell detection techniques such as flow cytometry, cell sorting, immunocytochemistry (*e.g*., staining with tissue specific or cell-marker specific antibodies), fluorescence activated cell sorting (FACS), magnetic activated cell sorting (MACS), by examination of the morphology of cells using light or confocal microscopy, or by measuring changes in gene expression using techniques well known in the art, such as PCR and gene expression profiling.

In one embodiment, placental stem cells may be sorted using a fluorescence activated cell sorter (FACS). Fluorescence activated cell sorting (FACS) is a well-known method for separating particles, including cells, based on the fluorescent properties of the particles (Kamarch, 1987, Methods Enzymol, 151:150-165). Laser excitation of fluorescent moieties in the individual particles results in a small electrical charge allowing electromagnetic separation of positive and negative particles from a mixture. In one embodiment, cell surface marker-specific antibodies or ligands are labeled with distinct fluorescent labels. Cells are processed through the cell sorter, allowing separation of cells based on their ability to bind to the antibodies used. FACS sorted particles may be directly deposited into individual wells of 96-well or 384-well plates to facilitate separation and cloning.

In another embodiment, magnetic beads can be used to separate cells. The cells may be sorted using a magnetic activated cell sorting (MACS) technique, a method for separating particles based on their ability to bind magnetic beads (0.5-100 µm diameter). A variety of useful modifications can be performed on the magnetic microspheres, including covalent addition of antibody that specifically recognizes a cell-solid phase surface molecule or hapten. A magnetic field is then applied, to physically manipulate the selected beads. The beads are then mixed with the cells to allow binding. Cells are then passed through a magnetic field to separate out cells having cell surface markers. These cells can then isolated and re-mixed with magnetic beads coupled to an antibody against additional cell surface markers. The cells are again passed through a magnetic field, isolating cells that bound both the antibodies. Such cells can then be diluted into separate dishes, such as microtiter dishes for clonal isolation.

In certain embodiments of the invention, the drained, exsanguinated placenta is cultured as a bioreactor, i.e., an ex vivo system for propagating placental stem cells. The number of propagated cells in the placental bioreactor can be maintained in a continuous state of balanced growth by periodically or continuously removing a portion of a culture medium or perfusion fluid that is introduced into the placental bioreactor, and from which the propagated cells may be recovered. Fresh medium or perfusion fluid is introduced at the same rate or in the same amount. To use the placenta as a bioreactor, it may be cultured for varying periods of time under sterile conditions by perfusion with perfusate solution as disclosed herein. In specific embodiments, the placenta is cultured for at least about 12, 24, 36, or 48 hours, or for 3-5 days, 6-10 days, or for one to two weeks. In a preferred embodiment, the placenta is cultured for about 48 hours. The cultured placenta is preferably "fed" periodically by the removal of spent media and the cells suspended in the media, and addition of fresh media. The cultured placenta is preferably stored under sterile conditions to reduce the possibility of contamination, and maintained under intermittent and periodic pressurization to create conditions that maintain an adequate supply of nutrients to the cells of the placenta. Perfusion and culture of the placenta can be both automated and computerized for efficiency and increased capacity.

In certain embodiments, placental stem cells are induced to propagate in the placenta bioreactor by introduction of nutrients, hormones, vitamins, growth factors, or any combination thereof, into the perfusion solution. Serum and other growth factors may be added to the propagation perfusion solution or medium. Growth factors are usually proteins and include, but are not limited to: cytokines, lymphokines, interferons, colony stimulating factors (CSFs), interferons, chemokines, and interleukins. Other growth factors that may be used include recombinant human hematopoietic growth factors including ligands, stem cell factors, thrombopoietin (Tpo), granulocyte colony-stimulating factor (G-CSF), leukemia inhibitory factor, basic fibroblast growth factor, placenta derived growth factor and epidermal growth factor.

### 5.4.1.4 Collection of Cells from the Placenta

As disclosed above, after exsanguination and perfusion of the placenta, embryonic-like stem cells migrate into the drained, empty microcirculation where, according to the methods of the invention, they are collected, preferably by collecting the effluent perfusate in a collecting vessel.

In preferred embodiments, cells cultured in the placenta are isolated from the effluent perfusate using techniques known by those skilled in the art, such as, for example, density gradient centrifugation, magnetic cell separation, FACS sorting, or other cell separation or sorting methods well known in the art, and sorted, for example, according to the scheme shown in FIG. 4.

In a specific embodiment, cells collected from the placenta can be recovered from the effluent perfusate by centrifugation at, e.g., about 5000 X g for about 15 minutes at room temperature, which separates cells from contaminating debris and platelets. The cell pellets are resuspended in, e.g., IMDM serum-free medium containing 2 U/ml heparin and 2 mM EDTA (GibcoBRL, NY). The total mononuclear cell fraction can be isolated using apheresis, preferably using a commercial collection kit such as LYMPHOPREP™ (Nycomed Pharma, Oslo, Norway). Cells are then counted using, e.g., a hemocytometer. Viability is typically evaluated by trypan blue exclusion. Isolation of cells is preferably achieved by "differential trypsinization," using a solution of, e.g., 0.05% trypsin with 0.2% EDTA (Sigma, St. Louis Mo.). Differential trypsinization is possible because fibroblastoid cells trypsinized in this manner detach from plastic cell culture surfaces within about five minutes, whereas other adherent populations require more than about 20-30 minutes incubation with trypsin. The detached fibroblastoid cells are harvested following trypsinization and trypsin neutralization, using Trypsin Neutralizing Solution (TNS, BioWhittaker). The cells can then be washed in a nutrient medium such as H.DMEM, and resuspended in, e.g. MSCGM.

In another embodiment, the isolated placenta is perfused for a period of time without collecting the perfusate, such that the placenta may be perfused for 2, 4, 6, 8, 10, 12, 20 or 24 hours or even days before the perfusate is collected. In such embodiments, for example, perfusion fluid can be introduced into the placenta and allowed to occupy the placental vasculature for a time prior to collection, or in the case of circulated perfusate, the perfusion fluid can be recirculated for such a time.

In another embodiment, cells cultured in the placenta bioreactor are isolated from the placenta by physically dissecting the cells away from the placenta.

In another embodiment, cells cultured in the placenta bioreactor are isolated from the placenta by dissociating the tissues of the placenta or a portion thereof, and recovering the cultured cells by art-known cell separation or sorting methods such as density gradient centrifugation, magnetic cell separation, FACS sorting, etc.

In a preferred embodiment, perfusion of the placenta and collection of effluent perfusate is repeated once or twice during the culturing of the placenta, until the number of recovered nucleated cells falls below 100 cells/ml. The perfusates are pooled and subjected to light centrifugation to remove platelets, debris and de-nucleated cell membranes. The nucleated cells are then isolated by Ficoll-Hypaque density gradient centrifugation and after washing, resuspended in HDMEM. For isolation of the adherent cells, aliquots of 5-10 x 10⁶ cells are placed in each of several T-75 flasks and cultured with commercially available Mesenchymal Stem Cell Growth Medium (MSCGM) obtained from BioWhittaker, and placed in a tissue culture incubator (37°C., 5% CO₂). After 10 to 15 days, non-adherent cells are removed from the flasks by washing with PBS. The PBS is then replaced by MSCGM. Flasks are preferably examined daily for the presence of various adherent cell types and in particular, for identification and expansion of clusters of fibroblastoid cells.

In other embodiments, the cells collected from the placenta are cryopreserved for use at a later time. Methods for cryopreservation of cells, such as stem cells, are well known in the art, for example, cryopreservation using the methods of Boyse et al. (U.S. Pat. No. 5,192,553, issued Mar. 9, 1993) or Hu et al. (WO 00/73421, published Dec. 7, 2000).

### 5.4.2 Isolation of Placental Stem Cells By Physical Disruption

Placental stem cells can be collected from a mammalian placenta by physical disruption, *e.g*., enzymatic digestion, of the organ. For example, the placenta, or a portion thereof, may be, *e.g*., crushed, sheared, minced, diced, chopped, macerated or the like, while in contact with the stem cell collection composition of the invention, and the tissue subsequently digested with one or more enzymes. The placenta, or a portion thereof, may also be physically disrupted and digested with one or more enzymes, and the resulting material then immersed in, or mixed into, the stem cell collection composition of the invention. Any method of physical disruption can be used, provided that the method of disruption leaves a plurality, more preferably a majority, and more preferably at least 60%, 70%, 80%, 90%, 95%, 98%, or 99% of the cells in said organ viable, as determined by, *e.g*., trypan blue exclusion.

The placenta can be dissected into components prior to physical disruption and/or enzymatic digestion and stem cell recovery. For example, placenta-derived stem cells can be obtained from the amniotic membrane, chorion, umbilical cord, placental cotyledons, or any combination thereof. Preferably, placenta-derived stem cells are obtained from placental tissue comprising amnion and chorion. Typically, placenta-derived stem cells can be obtained by disruption of a small block of placental tissue, e.g., a block of placental tissue that is 1, 2, 3, 4, 5, 6, 7, 8, 9, 10, 20, 30, 40, 50, 60, 70, 80, 90, 100,200, 300, 400, 500, 600, 700, 800, 900 or 1000 cubic millimeters in volume.

A preferred stem cell collection composition comprises one or more tissue-disruptive enzyme(s). Enzymatic digestion preferably uses a combination of enzymes, e.g., a combination of a matrix metalloprotease and a neutral protease, for example, a combination of collagenase and dispase. In one embodiment, enzymatic digestion of placental tissue uses a combination of a matrix metalloprotease, a neutral protease, and a mucolytic enzyme for digestion of hyaluronic acid, such as a combination of collagenase, dispase, and hyaluronidase or a combination of LIBERASE® (Boehringer Mannheim Corp., Indianapolis, Ind.) and hyaluronidase. Other enzymes that can be used to disrupt placenta tissue include papain, deoxyribonucleases, serine proteases, such as trypsin, chymotrypsin, or elastase. Serine proteases may be inhibited by alpha 2 microglobulin in serum and therefore the medium used for digestion is usually serum-free. EDTA and DNase are commonly used in enzyme digestion procedures to increase the efficiency of cell recovery. The digestate is preferably diluted so as to avoid trapping stem cells within the viscous digest.

Any combination of tissue digestion enzymes can be used. Typical concentrations for tissue digestion enzymes include, e.g., 50-200 U/mL for collagenase I and collagenase IV, 1-10 U/mL for dispase, and 10-100 U/mL for elastase. Proteases can be used in combination, that is, two or more proteases in the same digestion reaction, or can be used sequentially in order to liberate placental stem cells. For example, in one embodiment, a placenta, or part thereof, is digested first with an appropriate amount of collagenase I at 2 mg/ml for 30 minutes, followed by digestion with trypsin, 0.25%, for 10 minutes, at 37°C. Serine proteases are preferably used consecutively following use of other enzymes.

In another embodiment, the tissue can further be disrupted by the addition of a chelator, e.g., ethylene glycol bis(2-aminoethyl ether)-N,N,N'N'-tetraacetic acid (EGTA) or ethylenediaminetetraacetic acid (EDTA) to the stem cell collection composition comprising the stem cells, or to a solution in which the tissue is disrupted and/or digested prior to isolation of the stem cells with the stem cell collection composition.

It will be appreciated that where an entire placenta, or portion of a placenta comprising both fetal and maternal cells (for example, where the portion of the placenta comprises the chorion or cotyledons), the placenta-derived stem cells collected will comprise a mix of placenta-derived stem cells derived from both fetal and maternal sources. Where a portion of the placenta that comprises no, or a negligible number of, maternal cells (for example, amnion), the placenta-derived stem cells collected will comprise almost exclusively fetal placenta-derived stem cells.

Placental stem cells in the digested placental tissue can be isolated by, e.g., culturing of such cells with other cells in the digested tissue and isolating by differential trypsinization as described elsewhere herein. Alternatively, such cells can be purified using one or more antibodies to placental stem cell markers, followed by, e.g., magnetic bead separation. See Section 5.4.1.4.

### 5.5 COMBINATIONS OF UMBILICAL CORD BLOOD AND

### PLACENTAL STEM CELLS

The present invention provides umbilical cord blood comprising placental stem cells for use in a method of treating a disorder or condition caused by or associated with incomplete development of brain by using a combination of umbilical cord blood and placental stem cells. The placental stem cells can be stem cells contained within placental perfusate; placental stem cells initially collected from placental perfusate; placental stem cells collected from digestion of placental tissue; placental stem cells from placental perfusate or digestion of placental tissue, wherein the placental stem cells have been cultured in cell culture for a time sufficient for the placental stem cells to propagate for, e.g., 1, 2, 3, 4, 5, 6, 7, 8, 9, 10, 11, 12, 13, 14, 15, 16, 17, 18, 19, 20, 22, 24, 26, 28, 30, 32, 34, 36, 38 or 40 population doublings; or any combination of the foregoing. Placental stem cells to be combined with cord blood in the treatment of premature infants can be from a single placenta or a plurality of placentas.

The ratio of umbilical cord blood and placental stem cells can be determined according to the judgment of those of skill in the art. In certain embodiments, the ratio of umbilical cord blood to placental stem cells is 100,000,000:1, 50,000,000:1, 20,000,000:1, 10,000,000:1, 5,000,000:1, 2,000,000:1, 1,000,000:1, 500,000:1, 200,000:1, 100,000:1, 50,000:1, 20,000:1, 10,000:1, 5,000:1, 2,000:1, 1,000:1, 500:1, 200:1, 100:1, 50:1, 20:1, 10:1, 5:1, 2:1, 1:1; 1:2; 1:5; 1:10; 1:100; 1:200; 1:500; 1:1,000; 1:2,000; 1:5,000; 1:10,000; 1:20,000; 1:50,000; 1:100,000; 1:500,000; 1:1,000,000; 1:2,000,000; 1:5,000,000; 1:10,000,000; 1:20,000,000; 1:50,000,000; or 1:100,000,000, comparing numbers of total nucleated cells in each population, or comparing total numbers of stem cells in each population. In preferred embodiments, the ratio of umbilical cord blood to placental stem cells is between 20:1 and 1:20, or is 1:10, 1:5, 1:1, 5:1 or 10:1.

Placental stem cells and cord blood can be combined prior to administration to a premature infant, or can be administered separately.

### 5.5.1 Pharmaceutical Compositions

The present invention also encompasses pharmaceutical compositions comprising umbilical cord blood and placental stem cells, and a pharmaceutically-acceptable carrier.

In accordance with this embodiment, the combined umbilical cord blood and placental stem cells of the invention may be formulated as an injectable composition (e.g., WO 96/39101). In another embodiment, the combined umbilical cord blood and placental stem cells of the present invention may be formulated using polymerizable or cross linking hydrogels as described, e.g., in U.S. Patent Nos. 5,709,854; 5,516,532; 5,654,381.

In another embodiment, the invention provides for the maintenance of each stem cell population of the combined umbilical cord blood and placental stem cells, prior to administration to an individual, as separate pharmaceutical compositions to be administered sequentially or jointly to create the combined stem cell population in vivo. Each component may be stored and/or used in a separate container, e.g., a single bag (e.g., blood storage bag from Baxter, Becton-Dickinson, Medcep, National Hospital Products, Terumo, etc.) or separate syringe, which contains a single type of cell or cell population. In a specific embodiment, cord blood, or cord blood-derived nucleated or stem cells, are contained in one bag, and placental perfusate, or placental stem cells from placental perfusate, are contained in a second bag.

A population of placental stem cells can be enriched. In a specific embodiment, a population of cells comprising placental stem cells is enriched by removal of red blood cells and/or granulocytes according to standard methods, so that the remaining population of nucleated cells is enriched for placental stem cells relative to other cell types in placental perfusate. Such an enriched population of placental stem cells may be used unfrozen, or may be frozen for later use. If the population of cells is to be frozen, a standard cryopreservative (e.g., DMSO, glycerol, Epilife™ Cell Freezing Medium (Cascade Biologics) is added to the enriched population of cells before it is frozen.

The pharmaceutical compositions of the invention may comprise one or more agents that induce cell differentiation. In certain embodiments, an agent that induces differentiation includes, but is not limited to, Ca2+, EGF, α-FGF, β-FGF, PDGF, keratinocyte growth factor (KGF), TGF-β, cytokines (e.g., IL-1α, IL-1β, IFN-γ, TFN), retinoic acid, transferrin, hormones (e.g., androgen, estrogen, insulin, prolactin, triiodothyroxine, hydrocortisone, dexamethasone), sodium butyrate, TPA, DMSO, NMF, DMF, matrix elements (e.g., collagen, laminin, heparan sulfate, Matrigel™), or combinations thereof.

In another embodiment, the pharmaceutical composition of the invention may comprise one or more agents that suppress cellular differentiation. In certain embodiments, an agent that suppresses differentiation includes, but is not limited to, human Delta-1 and human Serrate-1 polypeptides (see, Sakano et al., U.S. Patent No. 6,337,387), leukemia inhibitory factor (LIF), stem cell factor, or combinations thereof.

The pharmaceutical compositions of the present invention may be treated prior to administration to an individual with a compound that modulates the activity of TNF-α. Preferred such compounds are disclosed in detail in, e.g., U.S. Application Publication No. 2003/0235909. Preferred compounds are referred to as IMiDs (immunomodulatory compounds) and SELCIDS® (Selective Cytokine Inhibitory Drugs), and particularly preferred compounds are available under the trade names ACTIMID™, REVIMID™ and REVLIMID™.

### 6. EXAMPLES

### 6.1 EXAMPLE 1: COLLECTION OF UMBILICAL CORD BLOOD AND PLACENTAL STEM CELLS

This example illustrates the collection of umbilical cord blood and placental stem cells.

### 6.1.1 Collection of Umbilical Cord Blood

Umbilical cord blood is collected using an umbilical cord blood collection kit such as described in U.S. Pat. Application Publication No. 2006/0060494, entitled "Cord Blood Collection Kit and Methods of Use Therefor."

Collection kits, containing standard chucks, sterile gauze pad, povidine iodine swabs, sterile alcohol pads, plastic umbilical cord blood clamps, slide clip or hemostat clamps and leak proof resealable bags or canisters are used.

The collection can be performed before the placenta is delivered (in utero collection), after the placenta is delivered (ex utero collection) or during a C-section, prior to delivery of placenta.

Briefly, the venipuncture site on the distal site on the umbilical cord is sterilized. The collection tubing leading from the large collection bag is clamped, the cap is removed from the needle, and the umbilical vein is cannulated with the bevel of the needle facing down toward the umbilical vein. The clamp is removed to allow the blood to flow and collection bag is lowered below the cannulation site to allow the blood to fill the collection bag by gravity.

When the blood flow stops, the venipuncture site is clamped and the needle is withdrawn from the umbilical vein. The collection bag is labelled and put into the insulated shipping container.

The placenta with the clamped umbilical cord blood is placed in the leak proof resealable bag and the bag is then properly sealed and labeled.

After collection, viability of umbilical cord blood cells is determined by hemocytometer after trypan blue staining.

### 6.1.2 Isolation of Placental Stem Cells

Following exsanguination of the umbilical cord and placenta, the placenta was placed in a sterile, insulated container at room temperature and delivered to the laboratory within 4 hours of birth. Placentas were discarded if, on inspection, they had evidence of physical damage such as fragmentation of the organ or avulsion of umbilical vessels. Placentas were maintained at room temperature (23±2°C) or refrigerated (4°C) in sterile containers for 2 to 20 hours. Periodically, the placentas were immersed and washed in sterile saline at 25±3°C to remove any visible surface blood or debris. The umbilical cod was transected approximately 5 cm from its insertion into the placenta and the umbilical vessels were cannulated with TEFLON® polymer or polypropylene catheters connected to a sterile fluid path allowing bi-directional perfusion of the placenta and recovery of the effluent fluid. The system employed in the present invention enabled all aspects of conditioning, perfusion and effluent collection to be performed under controlled ambient atmospheric conditions as well as real-time monitoring of intravascular pressure and flow rates, core and perfusate temperatures and recovered effluent volumes. A range of conditioning protocols were evaluated over a 24 hour post-partum period and the cellular composition of the effluent fluid was analyzed by flow cytometry, light microscopy and colony forming unit assays.

**Placental Conditioning:** The placenta was maintained under varying conditions in an attempt to simulate and sustain a physiologically compatible environment for the proliferation and recruitment of residual cells. The cannula was flushed with IMDM serum-free medium (GibcoBRL, NY) containing 2U/ml heparin (EJkins-Sinn, NJ). Perfusion of the placenta continued at a rate of 50 mL per minute until approximately 150 mL of perfusate was collected. This volume of perfusate was labeled "early fraction". Continued perfusion of the placenta at the same rate resulted in the collection of a second fraction of approximately 150 mL and was labeled "late fraction". During the course of the procedure, the placenta was gently massaged to aid in the perfusion process and assist in the recovery of cellular material. Effluent fluid was collected from the perfusion circuit by both gravity drainage and aspiration through the arterial cannula.

Placentas were obtained from delivery rooms along with cord blood after obtaining written parental consent, and were processed at room temperature within 12 to 24 hours after delivery. Before processing, the membranes were removed and the maternal site washed clean of residual blood. The umbilical vessels were cannulated with catheters made from 20 gauge Butterfly needles use for blood sample collection. Placentas were then perfused with heparinized (2U/mL) Dulbecco's modified Eagle Medium (H.DMEM) at the rate of 15 mL/minute for 10 minutes and the perfusates were collected from the maternal sites within one hour and the nucleated cells counted. The perfusion and collection procedures were repeated once or twice until the number of recovered nucleated cells fell below 100/mL. The perfusates were pooled and subjected to light centrifugation to remove platelets, debris and de-nucleated cell membranes. The nucleated cells were then isolated by Ficoll-Hypaque density gradient centrifugation and after washing, resuspended in H.DMEM. For isolation of the adherent cells, aliquots of 5-10x106 cells were placed in each of several T-75 flasks and cultured with commercially available Mesenchymal Stem Cell Growth Medium (MSCGM) obtained from BioWhittaker, and placed in a tissue culture incubator (37° C, 5% CO₂). After 10 to 15 days, the non-adherent cells were removed by washing with PBS, which was then replaced by MSCGM. The flasks were examined daily for the presence of various adherent cell types and in particular, for identification and expansion of clusters of fibroblastoid cells.

**Cell Recovery and Isolation:** Cells were recovered from the perfusates by centrifugation at 100 x g for 15 minutes at room temperature. This procedure served to separate cells from contaminating debris and platelets. The cell pellets were resuspended in IMDM serum-free medium containing 2 U/ml heparin and 2 mM EDTA (GibcoBRL, NY). The total mononuclear cell fraction was isolated using LYMPHOPREP™ (Nycomed Pharma, Oslo, Norway) according to the manufacturer's recommended procedure and the mononuclear cell fraction was resuspended. Cells were counted using a hemocytometer. Viability was evaluated by trypan blue exclusion. Isolation of mesenchymal cells was achieved by differential trypsinization using a solution of 0.05% trypsin with 0.2% EDTA (Sigma). Differential trypsinization was possible because fibroblastoid cells, including placental stem cells, detached from plastic surfaces within about five minutes whereas the other adherent populations required more than 20-30 minutes incubation. The detached fibroblastoid cells were harvested following trypsinization and trypsin neutralization, using Trypsin Neutralyzing Solution (TNS, BioWhitaker). The cells were washed in H.DMEM and resuspended in MSCGM. Flow cytometry was carried out using a Becton-Dickinson FACSCalibur instrument. FITC and PE labeled monoclonal antibodies, including antibodies for CD10, CD 34, CD44, CD45, CD54, CD90, SSEA3, and SSEA4, were purchased from Becton-Dickinson and Caltag laboratories (S. San Francisco, CA.), or other suppliers and SH2, SH3 and SH4 antibody producing hybridomas were obtained from the American Type Culture Collection, and reactivities of the monoclonal antibodies in their cultured supernatants were detected by FITC or PE labeled F(ab)'2 goat anti-mouse antibodies. Lineage differentiation was carried out using the commercially available induction and maintenance culture media (BioWhittaker), used as per manufacturer's instructions.

**Isolation of Placental Stem Cells:** Microscopic examination of adherent cells in the culture flasks revealed morphologically different cell types, including spindle-shaped cells, round cells with large nuclei and numerous perinuclear small vacuoles, and star-shaped cells with several projections, through one of which the cells were attached to the flask. Similar non-stem cells were observed in the culture of bone marrow, cord and peripheral blood; therefore, these cells were considered to be non-stem cell in nature. The fibroblastoid cells, appearing last as clusters, were candidates for being mesenchymal-like stem cells and were isolated by differential trypsinization and subcultured in secondary flasks. Post-trypsinization phase microscopy of the rounded cells revealed the cells to be highly granulated, and similar to bone marrow-derived MSC produced in the laboratory or purchased from commercial sources. When subcultured, the placental stem cells, in contrast to their earlier phase, adhered within hours, assumed a characteristic fibroblastoid shape, and formed a growth pattern identical to the reference bone marrow-derived MSC. Moreover, during subculturing and refeeding, the loosely bound mononuclear cells were washed out and the cultures remained homogeneous and devoid of any visible non-fibroblastoid cell contaminants.

**Flow Cytometry:** The expression of placental stem cell surface markers, including CD10, CD29, CD34, CD38, CD44, CD45, CD54, CD90, SSEA3 and SSEA4 was assessed by flow cytometry. Expression of OCT-4 and ABC-p was assessed by RT-PCR using known primers for these markers.

### 6.2 EXAMPLE 2: TREATMENT OF PREMATURE INFANTS WITH UMBILICAL CORD BLOOD AND PLACENTAL STEM CELLS

Six premature infants born at gestational age of between 23 weeks to 36 weeks exhibiting Respiratory Distress Syndrome (RDS) or Acute Respiratory Distress Syndrome (ARDS), anemia, intraventricular hemorrhage, necrotizing enterocolitis, retinopathy of prematurity, chronic lung disease (bronchopulmonary dysplasia), an infection, patent ductus arteriosus, apnea, low blood pressure, hyperbilirubinemia, incomplete development of lung, eye, immune system, brain, heart, liver or kidney are treated with umbilical cord blood and placental stem cells.

Umbilical cord blood is collected as described in Example 1 and placental stem cells are obtained by perfusion or by enzymatic digestion, as described in U.S. Patent Application Publication No. 2007/0275362, filed December 26, 2006. Umbilical cord blood and placental stem cells are combined at a ratio of 1:1. Umbilical cord blood and placental stem cells are characterized by FACS analysis. Umbilical cord cells and placental stem cells are injected intravenously to the premature infants at dosages of 1 x 10⁵ to 1 x 10⁶ CD34⁺ cells per kilogram body weight of the premature infants one week after delivery and two weeks after delivery.

Prior to and after administration of umbilical cord blood and placental stem cells, blood pressure, heart rate, respiratory rate, and counts of various blood cell types of the premature infants are measured.

### 6.3 EXAMPLE 3: CHARACTERIZATION OF CELLS FROM CORD BLOOD AND PLACENTAL PERFUSATE

The following experiments were performed to demonstrate the feasibility of producing a combined HPP and UCB from preterm placenta and to determine the cellular composition of the combined product as compared to cell isolated from term placenta.

Overall results show that (1) It is feasible to collect HPP and UCB from preterm placenta; (2) the total nuclear cell isolated from preterm placenta is comparable to that isolated from term placenta; (3) The TNC content of HPP/UCB isolated from preterm placenta is significantly higher than that shown in term placenta when normalized to similar weight; (4) the cellular composition of umbilical cord blood cells isolated from preterm placenta is different than that of term placenta; CD38⁺CD45⁺ cells are statistically significantly higher in term placenta compared to preterm placenta (p-value of 0.0146), while CD38⁻CD45⁻ cells are statistically significantly higher in preterm placenta (p-value of 0.0335); and (5) the cellular composition of HPP isolated from preterm placenta is not significantly different than term placenta.

It is feasible to generate HPP and UCB stem cells from preterm placenta in quantities which substantially exceeds the conventional methods to isolate UCB stem cells. The combined cellular product should be useful to treat complications associated with premature birth since it could be rich source of several progenitor cells with trophic capacity to protect endogenous cell death against hypoxia and differentiation capacity to form blood, angiogenic and neuronal cells in vivo.

### 6.3.1 Methods:

**Subjects:** Women awaiting elective caesarean sections were recruited in the antenatal clinic and those having spontaneous deliveries were recruited on the delivery suite.

**Cord blood and placenta collection:** The placenta was collected, and the umbilical cord was cut and clamped. As much cord blood as possible was drained from the umbilical cord. The cord was then clamped again to prevent further blood loss and the placenta and cord blood were immediately delivered to the lab.

**Laboratory processing:** On arrival in the lab, within 10-15 minutes from the time of delivery), the placenta was weighed and the placental membranes removed. The placenta and cord were assessed to see if they are suitable for perfusion, e.g., the cord is completely attached to the placenta and there is no sign of a tear and also that there are no deep tears in the placenta. The placenta is then covered with the preservation solution. The placenta is then placed in a refrigerator for 48 hours. A sample of cord blood was run immediately for Cell-Dyn and FACS analysis.

**Placental perfusion:** Placentas were perfused and the perfusate cryopreserved. Pressure controlled perfusions were accomplished using a Masterflex L/S 7523 programmable peristaltic pump. An umbilical catheter was inserted into each vein of the placenta umbilical cord and connected to a three-way stopcock. This assembly was then connected to a DPT100 disposable pressure transducer from Utah medical and then to Masterflex L/S 16 peristaltic pump tubing, which was in turn connected to a bag of injectible grade saline. Tubing from a blood collection bag was inserted into the umbilical vein to collect the placental blood. Labview software monitored the pressure of the perfusion, and the Masterflex pump was manually adjusted to the pressure desired. Perfusions were limited to 3 hours with volume and NOC testing completed after each hour of perfusion.

**Viability testing of placenta perfusate cells:** Bags containing human placental perfusate (HPP) were mixed thoroughly, and a small aliquot of the HPP was removed from the bag. The contaminating erythrocytes were then lysed by treating the sample with acetic acid. After erythrocyte cell lysis was complete, each sample was subjected to either Trypan Blue staining or counting by a Cell Dyne 3200. To determine percentage of viable cells, the numbers of intact cells in a microscopic field which exclude the uptake of Trypan Blue were determined in triplicate. The number of viable cells was divided by total cell number multiplied by 100.

**Flow Cytometry:** Flow cytometry studies were performed using HPP or UCB or combined cells using the following antibodies:

| Tube | FL1- FITC or Alexa488 | FL2- PE | FL3 PerCP | FL4 APC or Alexa647 | FL5 PE-CY7 APC-CY7 or APC Alexa750 | FL6 Pacific Blue |
|---|---|---|---|---|---|---|
| 1 | PS | 235a | 7AAD | 38 | 34 | 45 |
| 2 | 90 | 133 | 69 | 38 | 34 | 45 |
| 3 | 44 | 117 | 7AAD | 105 | 34 | 45 |

Cells were washed with buffer, and then re-suspended at a set concentration range (i.e. 1x10⁶ live cells per 100 µL) in buffer. Cells were then stained with fluorescence-conjugated antibodies, incubated, and then washed with buffer to remove excess antibodies. Stained cells were tested on a flow cytometer to determine positive or negative expression of the markers of interest.

### 6.3.2 Results

Pre-term and term placentas were found to have significantly different weights (average of 345 g and 731 g, respectively; p = 0.0001). The viability of cells from pre-term and term placentas was not found to differ significantly (viability of 93.13% and 90.84%, respectively; p = 0.0724). However, the total nucleated cord blood cells obtainable from pre-term did differ significantly (p = 0.0001). A mean of 5.277 x 10⁷ cells were obtained from pre-term placentas, and a mean of 1.9498 x 10⁸ cells were obtained from term placentas. Similarly, the total nucleated perfusate cells obtainable from pre-term did differ significantly (p = 0.0016). A mean of 1.28 x 10⁷ cells were obtained from pre-term placentas, and a mean of 3.76 x 10⁷ cells were obtained from term placentas. Thus, the number of nucleated cells obtainable per gram of placental tissue was significantly greater for pre-term placentas than term placentas (p = 0.0001). The total perfusate + cord blood nucleated cells that can be obtained from a pre-term placenta is 1.79 x 10⁸, compared to This is extremely significantly different compared with a term placenta of 5.71 x 10⁸ for term placenta (p = 0.0001). No significant difference in the number of combined total nucleated cells per gram of placental tissue was found.

### 6.3.3 Flow Cytometry

No statistically significant difference between the percentage of CD34⁺ cells, CD38⁻CD45⁺ cells, or CD38⁺CD45⁻ cells in the cord blood from pre-term and term placentas was found. Significantly higher numbers of CD38⁺CD45⁺ cells were found in term placenta (16.68% of TNC vs. 2.0% TNC in pre-term), and significantly higher numbers of CD38⁻CD45⁻ cells are found in pre-term placenta (56.52% vs. 24.58% for term placenta). No statistically significant difference in the percentage of perfusate-derived CD38⁻CD45⁺ cells, CD38⁺CD45⁻ cells, CD38⁺CD45⁺ cells or CD38⁻CD45⁻ cells was found between pre-term and term placentas.

## Claims

1. Umbilical cord blood comprising placental stem cells for use in a method of treating a disorder or condition in a premature infant, wherein said disorder or condition is caused by or associated with incomplete development of the brain, wherein said placental stem cells comprise cells that are CD34⁻, CD73⁺, CD105⁺, and CD200⁺.

2. The umbilical cord blood for use of claim 1, further comprising a blood additive, wherein said blood additive is erythropoietin, an iron supplement, a vitamin, or red blood cells from a source other than cord blood.

3. The umbilical cord blood for use of claim 2, wherein said blood additive is erythropoietin.

4. The umbilical cord blood for use of claim 3, wherein said erythropoietin is recombinant erythropoietin genetically engineered to increase serum half-life compared to native erythropoietin.

5. The umbilical cord blood for use of claim 2, wherein said blood additive is a vitamin.

6. The umbilical cord blood for use of claim 5, wherein said vitamin is riboflavin (vitamin B2), pyridoxine (vitamin B6), folic acid, vitamin B12, or vitamin E.

7. The umbilical cord blood for use of claim 2, wherein the blood additive is an iron supplement.

8. The umbilical cord blood for use of claim 7, wherein said iron supplement is elemental iron.

9. The umbilical cord blood for use of claim 2, wherein said blood additive is red blood cells not obtained from cord blood.

10. The umbilical cord blood for use of claim 9, wherein said red blood cells have been irradiated with at least 2500 cGy of radiation, or wherein said red blood cells have been leukodepleted.

11. The umbilical cord blood for use of claim 1, wherein said premature infant has undergone from 23 to 25 weeks of gestation at birth, from 26 to 29 weeks of gestation at birth, from 30 to 33 weeks of gestation at birth, or from 34 to 37 weeks of gestation at birth.

12. The umbilical cord blood for use of claim 1, wherein said premature infant weighs 800 grams or more at birth, 500 grams to 800 grams at birth, or less than 500 grams at birth.

13. The umbilical cord blood for use of claim 1, wherein said umbilical cord blood is autologous to the premature infant.

14. The umbilical cord blood for use of claim 1, wherein the placental stem cells are autologous to the premature infant.

15. The umbilical cord blood for use of claim 1, wherein said umbilical cord blood is obtained from a post-partum mammalian placenta of a full-term birth.

16. The umbilical cord blood for use of claim 1, wherein said umbilical cord blood is obtained from a post-partum mammalian placenta of a premature birth.

17. The umbilical cord blood for use of claim 16, wherein said placenta is the placenta of said premature infant, the placenta of an infant born at 26 to 29 weeks of gestation, the placenta of an infant born at 30 to 33 weeks of gestation, or the placenta of an infant born at 34 to 37 weeks of gestation.

18. The umbilical cord blood for use of claim 1, wherein said umbilical cord blood is obtained from a cord blood bank.

19. The umbilical cord blood for use of claim 1, wherein said placental stem cells are stem cells isolated from placental perfusate prior to said use.

20. The umbilical cord blood for use of claim 1, wherein said placental stem cells are stem cells contained within placental perfusate.

21. The umbilical cord blood for use of claim 1, wherein said placental stem cells are obtained from a post-partum mammalian placenta of a full-term birth, from a post-partum mammalian placenta of a premature birth, or from a placenta of said premature infant.

22. The umbilical cord blood for use of claim 1, wherein said placental stem cells are obtained from a placenta of an infant born at 23 to 25 weeks of gestation, from a placenta of an infant born at 26 to 29 weeks of gestation, from a placenta of an infant born at 30 to 33 weeks of gestation, or from a placenta of an infant born at 34 to 37 weeks of gestation.

23. The umbilical cord blood for use of claim 1, wherein said umbilical cord blood or said placental stem cells are not immunotyped prior to said use.

24. The umbilical cord blood for use of claim 1, wherein said umbilical cord blood is prepared to be administered once after birth of the premature infant, a plurality of times after birth of the premature infant, within one hour after birth of the premature infant, within 12 hours after birth of the premature infant, within 24 hours after birth of the premature infant, or within one week after birth of the premature infant.

25. The umbilical cord blood for use of claim 1, wherein said umbilical cord blood comprises 1 x 10⁵ to 1 x 10⁶ CD34⁺ cells per kilogram body weight of said premature infant.

26. The umbilical cord blood for use of claim 1, wherein said placental stem cells and said umbilical cord blood together comprises 1 x 10⁵ to 1 x 10⁶ CD34⁺ cells per kilogram body weight of said premature infant.

27. The umbilical cord blood for use of claim 1, wherein said umbilical cord blood is prepared to be administered by intravenous injection.

28. The umbilical cord blood for use of claim 1, wherein said umbilical cord blood is prepared to be administered within 1 hour after birth, within 12 hours after birth, within 2 days after birth, or within 2 weeks after birth.

## Patentansprüche

1. Nabelschnurblut umfassend Plazentastammzellen für die Verwendung in einem Verfahren zur Behandlung einer Erkrankung oder eines Zustands bei einem Frühgeborenen, wobei die Erkrankung oder der Zustand durch unvollständige Entwicklung des Gehirns verursacht oder damit verbunden ist, wobei die Plazentastammzellen Zellen umfassen, die CD34⁻, CD73⁺, CD105⁺ und CD200⁺ sind.

2. Nabelschnurblut für die Verwendung nach Anspruch 1, weiter umfassend einen Blutzusatz, wobei der Blutzusatz Erythropoietin, ein Eisenpräparat, ein Vitamin oder rote Blutkörperchen aus einer Quelle außer Nabelschnurblut ist.

3. Nabelschnurblut für die Verwendung nach Anspruch 2, wobei der Blutzusatz Erythropoietin ist.

4. Nabelschnurblut für die Verwendung nach Anspruch 3, wobei das Erythropoietin rekombinantes Erythropoietin ist, das genetisch verändert ist, um die Serum-Halbwertszeit verglichen mit natürlichem Erythropoietin zu erhöhen.

5. Nabelschnurblut für die Verwendung nach Anspruch 2, wobei der Blutzusatz ein Vitamin ist.

6. Nabelschnurblut für die Verwendung nach Anspruch 5, wobei das Vitamin Riboflavin (Vitamin B2), Pyridoxin (Vitamin B6), Folsäure, Vitamin B12 oder Vitamin E ist.

7. Nabelschnurblut für die Verwendung nach Anspruch 2, wobei der Blutzusatz ein Eisenpräparat ist.

8. Nabelschnurblut für die Verwendung nach Anspruch 7, wobei das Eisenpräparat elementares Eisen ist.

9. Nabelschnurblut für die Verwendung nach Anspruch 2, wobei der Blutzusatz rote Blutkörperchen ist, die nicht aus Nabelschnurblut gewonnen wurden.

10. Nabelschnurblut für die Verwendung nach Anspruch 9, wobei die roten Blutkörperchen mit mindestens 2500 cGy an Strahlung bestrahlt worden sind, oder wobei die roten Blutkörperchen an Leukozyten verringert worden sind.

11. Nabelschnurblut für die Verwendung nach Anspruch 1, wobei das Frühgeborene 23 bis 25 Wochen Gestation vor der Geburt, 26 bis 29 Wochen Gestation vor der Geburt, 30 bis 33 Wochen Gestation vor der Geburt oder 34 bis 37 Wochen Gestation vor der Geburt erfahren hat.

12. Nabelschnurblut für die Verwendung nach Anspruch 1, wobei das Frühgeborene 800 Gramm oder mehr bei der Geburt, 500 Gramm bis 800 Gramm bei der Geburt oder weniger als 500 Gramm bei der Geburt wiegt.

13. Nabelschnurblut für die Verwendung nach Anspruch 1, wobei das Nabelschnurblut autolog für das Frühgeborene ist.

14. Nabelschnurblut für die Verwendung nach Anspruch 1, wobei die Plazentastammzellen autolog für das Frühgeborene sind.

15. Nabelschnurblut für die Verwendung nach Anspruch 1, wobei das Nabelschnurblut von einer postpartalen Säugetierplazenta einer voll ausgetragenen Geburt erhalten ist.

16. Nabelschnurblut für die Verwendung nach Anspruch 1, wobei das Nabelschnurblut von einer postpartalen Säugetierplazenta einer Frühgeburt erhalten ist.

17. Nabelschnurblut für die Verwendung nach Anspruch 16, wobei die Plazenta die Plazenta des Frühgeborenen, die Plazenta eines nach 26 bis 29 Wochen der Gestation geborenen Säuglings, die Plazenta eines nach 30 bis 33 Wochen der Gestation geboren Säuglings oder die Plazenta eines nach 34 bis 37 Wochen der Gestation geborenen Säuglings ist.

18. Nabelschnurblut für die Verwendung nach Anspruch 1, wobei das Nabelschnurblut von einer Nabelschnur-Blutbank erhalten ist.

19. Nabelschnurblut für die Verwendung nach Anspruch 1, wobei die Plazentastammzellen Stammzellen sind, die vor der Verwendung aus einem Plazentaperfusat isoliert wurden.

20. Nabelschnurblut für die Verwendung nach Anspruch 1, wobei die Plazentastammzellen Stammzellen sind, die in dem Plazentaperfusat enthalten sind.

21. Nabelschnurblut für die Verwendung nach Anspruch 1, wobei die Plazentastammzellen von einer postpartalen Säugetierplazenta einer voll ausgetragenen Geburt, von einer postpartalen Säugetierplazenta einer Frühgeburt oder von einer Plazenta des Frühgeborenen erhalten werden.

22. Nabelschnurblut für die Verwendung nach Anspruch 1, wobei die Plazentastammzellen von einer Plazenta eines Säuglings nach 23 bis 25 Wochen der Gestation, von einer Plazenta eines Säuglings nach 26 bis 29 Wochen der Gestation, von einer Plazenta eines Säuglings nach 30 bis 33 Wochen der Gestation oder von einer Plazenta eines Säuglings nach 34 bis 37 Wochen der Gestation erhalten werden.

23. Nabelschnurblut für die Verwendung nach Anspruch 1, wobei das Nabelschnurblut oder die Plazentastammzellen vor der Verwendung nicht immunotypisiert sind.

24. Nabelschnurblut für die Verwendung nach Anspruch 1, wobei das Nabelschnurblut vorbereitet ist, um einmal nach der Geburt des Frühgeborenen, mehrmals nach der Geburt des Frühgeborenen, innerhalb einer Stunde nach der Geburt des Frühgeborenen, innerhalb von 12 Stunden nach der Geburt des Frühgeborenen, innerhalb von 24 Stunden nach der Geburt des Frühgeborenen oder innerhalb einer Woche nach der Geburt des Frühgeborenen verabreicht zu werden.

25. Nabelschnurblut für die Verwendung nach Anspruch 1, wobei das Nabelschnurblut 1 x 10⁵ bis 1 x 10⁶ CD34⁺ Zellen pro Kilogramm Körpergewicht des Frühgeborenen umfasst.

26. Nabelschnurblut für die Verwendung nach Anspruch 1, wobei die Plazentastammzellen und das Nabelschnurblut zusammen 1 x 10⁵ bis 1 x 10⁶ CD34+ Zellen pro Kilogramm Körpergewicht des Frühgeborenen umfassen.

27. Nabelschnurblut für die Verwendung nach Anspruch 1, wobei das Nabelschnurblut vorbereitet ist, um durch intravenöse Injektion verabreicht zu werden.

28. Nabelschnurblut für die Verwendung nach Anspruch 1, wobei das Nabelschnurblut vorbereitet ist, um innerhalb einer Stunde nach der Geburt, innerhalb von 12 Stunden nach der Geburt, innerhalb von 2 Tagen nach der Geburt oder innerhalb von 2 Wochen nach der Geburt verabreicht zu werden.

## Revendications

1. Sang de cordon ombilical comprenant des cellules souches placentaires destiné à être utilisé dans une méthode de traitement d'un trouble ou d'un état chez un nourrisson prématuré, dans lequel ledit trouble ou état est provoqué par ou associé à un développement incomplet du cerveau, dans lequel lesdites cellules souches placentaires comprennent des cellules qui sont des CD34 , des CD73+, des CD105+, et des CD200+.

2. Sang de cordon ombilical destiné à être utilisé selon la revendication 1, comprenant en outre un additif sanguin, dans lequel ledit additif sanguin est de l' érythropoïétine, un complément en fer, une vitamine, ou des érythrocytes provenant d'une source autre que du sang de cordon.

3. Sang de cordon ombilical destiné à être utilisé selon la revendication 2, dans lequel ledit additif sanguin est de l'érythropoïétine.

4. Sang de cordon ombilical destiné à être utilisé selon la revendication 3, dans lequel ladite érythropoïétine est de l'érythropoïétine de recombinaison génétiquement modifiée afin d'augmenter la demi-vie sérique en comparaison avec de l'érythropoïétine native.

5. Sang de cordon ombilical destiné à être utilisé selon la revendication 2, dans lequel ledit additif sanguin est une vitamine.

6. Sang de cordon ombilical destiné à être utilisé selon la revendication 5, dans lequel ladite vitamine est la riboflavine (vitamine B2), la pyridoxine (vitamine B6), l'acide folique, la vitamine B 12, ou la vitamine E.

7. Sang de cordon ombilical destiné à être utilisé selon la revendication 2, dans lequel l'additif sanguin est un complément en fer.

8. Sang de cordon ombilical destiné à être utilisé selon la revendication 7, dans lequel ledit complément en fer est du fer élémentaire.

9. Sang de cordon ombilical destiné à être utilisé selon la revendication 2, dans lequel ledit additif sanguin est des érythrocytes non obtenus à partir de sang de cordon.

10. Sang de cordon ombilical destiné à être utilisé selon la revendication 9, dans lequel lesdits érythrocytes ont été irradiés avec au moins 2 500 cGy de rayonnements, ou dans lequel lesdits érythrocytes ont été appauvris en leucocytes.

11. Sang de cordon ombilical destiné à être utilisé selon la revendication 1, dans lequel ledit nourrisson prématuré a subi de 23 à 25 semaines de gestation à la naissance, de 26 à 29 semaines de gestation à la naissance, de 30 à 33 semaines de gestation à la naissance, ou de 34 à 37 semaines de gestation à la naissance.

12. Sang de cordon ombilical destiné à être utilisé selon la revendication 1, dans lequel ledit nourrisson prématuré pèse 800 grammes ou plus à la naissance, de 500 grammes à 800 grammes à la naissance, ou moins de 500 grammes à la naissance.

13. Sang de cordon ombilical destiné à être utilisé selon la revendication 1, ledit sang de cordon ombilical étant autologue vis-à-vis du nourrisson prématuré.

14. Sang de cordon ombilical destiné à être utilisé selon la revendication 1, dans lequel les cellules souches placentaires sont autologues vis-à-vis du nourrisson prématuré.

15. Sang de cordon ombilical destiné à être utilisé selon la revendication 1, ledit sang de cordon ombilical étant obtenu à partir d'un placenta mammalien du post-partum d'une naissance à terme.

16. Sang de cordon ombilical destiné à être utilisé selon la revendication 1, ledit sang de cordon ombilical étant obtenu à partir d'un placenta mammalien du post-partum d'une naissance prématurée.

17. Sang de cordon ombilical destiné à être utilisé selon la revendication 16, dans lequel ledit placenta est le placenta dudit nourrisson prématuré, le placenta d'un nourrisson né à un terme de 26 à 29 semaines de gestation, le placenta d'un nourrisson né à un terme de 30 à 33 semaines de gestation, ou le placenta d'un nourrisson né à un terme de 34 à 37 semaines de gestation.

18. Sang de cordon ombilical destiné à être utilisé selon la revendication 1, ledit sang de cordon ombilical étant obtenu à partir d'une banque de sang de cordon.

19. Sang de cordon ombilical destiné à être utilisé selon la revendication 1, dans lequel lesdites cellules souches placentaires sont des cellules souches isolées à partir d'un perfusat placentaire préalablement à ladite utilisation.

20. Sang de cordon ombilical destiné à être utilisé selon la revendication 1, dans lequel lesdites cellules souches placentaires sont des cellules souches contenues au sein d'un perfusat placentaire.

21. Sang de cordon ombilical destiné à être utilisé selon la revendication 1, dans lequel lesdites cellules souches placentaires sont obtenues à partir d'un placenta mammalien du post-partum d'une naissance à terme, d'un placenta mammalien du post-partum d'une naissance prématurée, ou d'un placenta dudit nourrisson prématuré.

22. Sang de cordon ombilical destiné à être utilisé selon la revendication 1, dans lequel lesdites cellules souches placentaires sont obtenues à partir d'un placenta d'un nourrisson né à un terme de 23 à 25 semaines de gestation, d'un placenta d'un nourrisson né à un terme de 26 à 29 semaines de gestation, d'un placenta d'un nourrisson né à un terme de 30 à 33 semaines de gestation, ou d'un placenta d'un nourrisson né à un terme de 34 à 37 semaines de gestation.

23. Sang de cordon ombilical destiné à être utilisé selon la revendication 1, ledit sang de cordon ombilical n'étant pas immunotypé ou lesdites cellules souches placentaires n'étant pas immunotypées préalablement à ladite utilisation.

24. Sang de cordon ombilical destiné à être utilisé selon la revendication 1, ledit sang de cordon ombilical étant préparé afin d'être administré une fois après la naissance du nourrisson prématuré, une pluralité de fois après la naissance du nourrisson prématuré, dans l'heure suivant la naissance du nourrisson prématuré, dans les 12 heures suivant la naissance du nourrisson prématuré, dans les 24 heures suivant la naissance du nourrisson prématuré, ou dans la semaine suivant la naissance du nourrisson prématuré.

25. Sang de cordon ombilical destiné à être utilisé selon la revendication 1, ledit sang de cordon ombilical comprenant de 1 x 105 à 1 x 106 cellules CD34+ par kilogramme de poids corporel dudit nourrisson prématuré.

26. Sang de cordon ombilical destiné à être utilisé selon la revendication 1, dans lequel lesdites cellules souches placentaires et ledit sang de cordon ombilical comprennent ensemble de 1 x 105 à 1 x 106 cellules CD34+ par kilogramme de poids corporel dudit nourrisson prématuré.

27. Sang de cordon ombilical destiné à être utilisé selon la revendication 1, ledit sang de cordon ombilical étant préparé afin d'être administré par injection intraveineuse.

28. Sang de cordon ombilical destiné à être utilisé selon la revendication 1, ledit sang de cordon ombilical étant préparé afin d'être administré dans l'heure suivant la naissance, dans les 12 heures suivant la naissance, dans les 2 jours suivant la naissance, ou dans les 2 semaines après la naissance.
